(19) Europäisches Patentamt
European Patent Office
Office européen des brevets

(11) EP 0 998 261 B1

(12) FASCICULE DE BREVET EUROPEEN

(45) Date de publication et mention de la délivrance du brevet:
**23.04.2003 Bulletin 2003/17**

(21) Numéro de dépôt: **99907685.4**

(22) Date de dépôt: **11.03.1999**

(51) Int Cl.$^7$: **A61K 7/13**

(86) Numéro de dépôt international:
**PCT/FR99/00542**

(87) Numéro de publication internationale:
**WO 99/048466 (30.09.1999 Gazette 1999/39)**

# (54) COMPOSITION DE TEINTURE D'OXYDATION DES FIBRES KERATINIQUES CONTENANT UN DERIVE AZO DE 3-AMINO PYRIDINE ET PROCEDE DE TEINTURE METTANT EN OEUVRE CETTE COMPOSITION

OXIDATIONSFÄRBEZUSAMMENSETZUNG FÜR KERATINISCHE FASERN, DIE AZODERIVATE VON 3-AMINO-PYRIDIN ENTHÄLT, UND FÄRBEVERFAHREN

OXIDATION DYEING COMPOSITION FOR KERATINOUS FIBRES CONTAINING A 3-AMINOPYRIDINE AZO DERIVATIVE AND DYEING METHOD USING SAID COMPOSITION

(84) Etats contractants désignés:
**AT BE CH CY DE DK ES FI FR GB GR IE IT LI LU MC NL PT SE**

(30) Priorité: **20.03.1998 FR 9803453**

(43) Date de publication de la demande:
**10.05.2000 Bulletin 2000/19**

(73) Titulaire: **L'OREAL**
**75008 Paris (FR)**

(72) Inventeurs:
- **LANG, Gérard**
  **F-95390 Saint Prix (FR)**
- **COTTERET, Jean**
  **F-78480 Verneuil-sur-Seine (FR)**
- **MAUBRU, Mireille**
  **F-78400 Chatou (FR)**

(74) Mandataire: **Goulard, Sophie et al**
**L'OREAL-DPI**
**6 rue Bertrand Sincholle**
**92585 Clichy Cedex (FR)**

(56) Documents cités:
**EP-A- 0 739 622**
**WO-A-97/39727**
**DE-A- 4 241 173**
**US-A- 4 025 301**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 998 261 B1

Printed by Jouve, 75001 PARIS (FR)

## Description

L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation, au moins un dérivé de l'amino-3 pyridine à titre de colorant direct, et au moins un méta-aminophénol substitué à titre de coupleur, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

Il est également connu que pour faire encore varier les nuances obtenues et leur donner des reflets, on peut utiliser, en association avec les précurseurs de colorants d'oxydation et les coupleurs, des colorants directs, c'est à dire des substances colorées qui apportent une coloration en l'absence d'agent oxydant.

Ces colorants directs appartiennent pour leur très grande majorité à la famille des composés nitrés de la série benzénique et ont l'inconvénient, lorsqu'ils sont incorporés dans des compositions tinctoriales, de conduire à des colorations présentant une ténacité insuffisante, en particulier vis-à-vis des shampooings.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet FR-A-2 285 851, des compositions pour la teinture d'oxydation des fibres kératiniques contenant l'association d'une base d'oxydation benzénique, d'un colorant direct de la famille des 3-amino pyridines et d'un méta-aminophénol non substitué à titre de coupleur. Cependant les colorations obtenues en mettant en oeuvre de telles compositions ne sont pas entièrement satisfaisantes, notamment du point de vue de leur chromaticité et de leur ténacité.

Or, la demanderesse vient maintenant de découvrir qu'il est possible d'obtenir de nouvelles teintures, capables de conduire à des colorations puissantes et chromatiques, peu sélectives et résistant bien aux diverses agressions que peuvent subir les fibres, en associant au moins une base d'oxydation, au moins un dérivé de 3-amino pyridine convenablement sélectionné à titre de colorant direct, et au moins un dérivé de méta-aminophénol convenablement sélectionné.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation,
- à titre de colorant direct, au moins un dérivé de 3-amino pyridine choisi parmi les composés de formule (I) suivante :

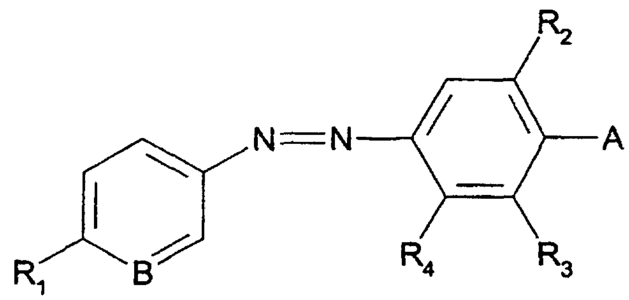

(I)

dans laquelle :

- B représente un groupement de formules (Ia) ou (Ib) suivantes :

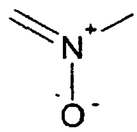

(Ia)

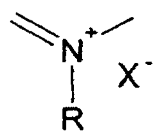

(Ib)

- R représente un radical alkyle en $C_1$-$C_4$ ;
- $R_1$ représente un atome d'hydrogène ou d'halogène tel que le chlore, le brome ou le fluor, un radical alkyle en $C_1$-$C_4$ ou alcoxy en $C_1$-$C_4$ ;
- $R_2$ représente un atome d'hydrogène, un radical alkyle en $C_1$-$C_4$ ou alcoxy en $C_1$-$C_4$ ;
- $R_4$ représente un atome d'hydrogène ou d'halogène tel que le chlore, le brome ou le fluor, un radical alkyle en $C_1$-$C_4$, nitro, amino ou acyl($C_1$-$C_4$)amino ;
- $R_3$ représente un atome d'hydrogène ou bien $R_4$ et $R_3$ forment un cycle insaturé à 6 chaînons portant un substituant hydroxyle chélaté avec un des atomes d'azote de la double liaison azoïque :
- A représente un reste $-NR_5R_6$ dans lequel $R_5$ représente un atome d'hydrogène, un radical alkyle en $C_1$-$C_4$, monohydroxyalkyle en $C_1$-$C_4$ ou polyhydroxyalkyle en $C_2$-$C_4$ ; $R_6$ représente un atome d'hydrogène, un radical alkyle en $C_1$-$C_4$, monohydroxyalkyle en $C_1$-$C_4$ ou polyhydroxyalkyle en $C_2$-$C_4$, un cycle phényle ou un radical $-CH_2-SO_3Na$ ;
- $X^-$ représente un anion monovalent ou divalent et est de préférence choisi parmi un atome d'halogène tel que le chlore, le brome, le fluor ou l'iode, un hydroxyde, un hydrogènesulfate, ou un alkyl($C_1$-$C_6$)sulfate tel que par exemple un méthylsulfate ou un éthylsulfate, et
- au moins un coupleur choisi parmi les dérivés de méta-aminophénol de formule (II) suivante, et leurs sels d'addition avec un acide :

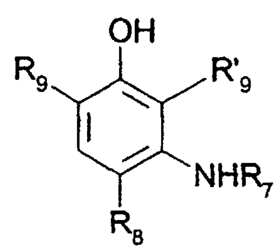

(II)

dans laquelle :

- $R_7$ représente un atome d'hydrogène, un radical alkyle en $C_1$-$C_4$, monohydroxyalkyle en $C_1$-$C_4$, polyhydroxyalkyle en $C_2$-$C_4$ ou monoaminoalkyle en $C_1$-$C_4$ ;
- $R_8$ représente un atome d'hydrogène, un radical alkyle en $C_1$-$C_4$, alcoxy en $C_1$-$C_4$ ou un atome d'halogène choisi parmi le chlore, le brome ou le fluor,
- $R_9$ et $R'_9$, identiques ou différents, représentent un atome d'hydrogène ou d'halogène, un radical alkyle en $C_1$-$C_4$,

alcoxy en $C_1$-$C_4$, monohydroxyalkyle en $C_1$-$C_4$, polyhydroxyalkyle en $C_2$-$C_4$, monohydroxyalcoxy en $C_1$-$C_4$ ou polyhydroxyalcoxy en $C_2$-$C_4$ ;

étant entendu qu'au moins un des radicaux $R_7$, $R_8$, $R_9$ et $R'_9$ est différent d'un atome d'hydrogène.

La composition tinctoriale conforme à l'invention conduit à des colorations puissantes, chromatiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux. Ces propriétés sont particulièrement remarquables en ce qui concerne la chromaticité.

L'invention a également pour objet un procédé de teinture d'oxydation des fibres kératiniques mettant en oeuvre cette composition tinctoriale.

La nature de la ou des bases d'oxydation utilisées dans la composition tinctoriale prête à l'emploi n'est pas critique. Elles peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (III) suivante et leurs sels d'addition avec un acide :

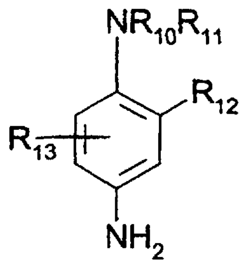
(III)

dans laquelle :

- $R_{10}$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;
- $R_{11}$ représente un atome d'hydrogène, un radical aikyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_{12}$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$, hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,
- $R_{13}$ représente un atome d'hydrogène, d'halogène ou un radical alkyle en $C_1$-$C_4$.

Parmi les groupements azotés de la formule (III) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les paraphénylènediamines de formule (III) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N, N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (III) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (IV) suivante, et leurs sels d'addition avec un acide :

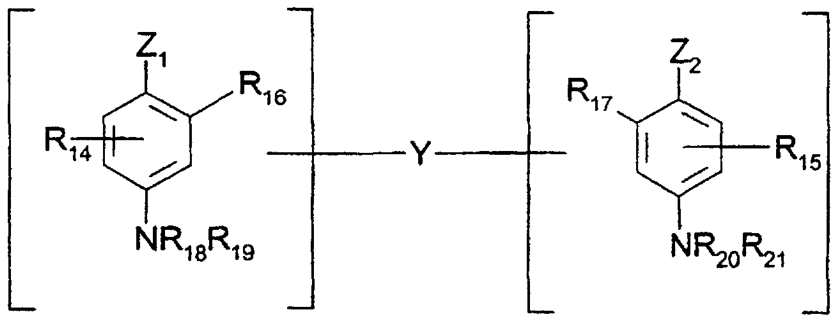

(II)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_{14}$ et $R_{15}$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_{16}$, $R_{17}$, $R_{18}$, $R_{19}$ $R_{20}$ et $R_{21}$, identiques ou différents, représentent un atome d'hydrogène, un bras de liaison Y ou un radical alkyle en $C_1$-$C_4$ ;

étant entendu que les composés de formule (IV) ne comportent qu'un seul bras de liaison Y par molécule.

Parmi les groupements azotés de la formule (IV) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les bases doubles de formules (IV) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (IV), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés de formule (V) suivante, et leurs sels d'addition avec un acide :

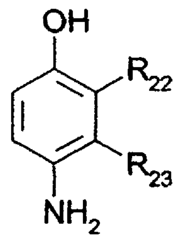

(V)

dans laquelle :

- $R_{22}$ représente un atome d'hydrogène ou d'halogène, un radical alkyle en $C_1$-$C_4$, monohydroxyalkyle en $C_1$-$C_4$, alcoxy($C_1$-$C_4$)alkyle($C_1$-$C_4$), aminoalkyle en $C_1$-$C_4$ ou hydroxyalkyl($C_1$-$C_4$)aminoalkyle en $C_1$-$C_4$,
- $R_{23}$ représente un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$);

étant entendu qu'au moins un des radicaux $R_{22}$ et $R_{23}$ représente un atome d'hydrogène.

Parmi les para-aminophénols de formule (V) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolopyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-Amino-pyrazolo[1,5-a]pyrimidin-7-yl)-(2-hydroxy-éthyl)-amino]-éthanol, le 2-[(7-Amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation représentent de préférence de 0,0005 à 12 % en poids environ du poids total de la composition tinctoriale conforme à l'invention, et encore plus préférentiellement de 0,005 à 6 % en poids environ de

ce poids.

Le ou les dérivés de 3-amino pyridine de formule (I) conformes à l'invention sont de préférence choisis parmi :

- le méthosulfate de diméthylamino-4' benzène azo-1' : 3-méthyl-1-pyridinium de formule :

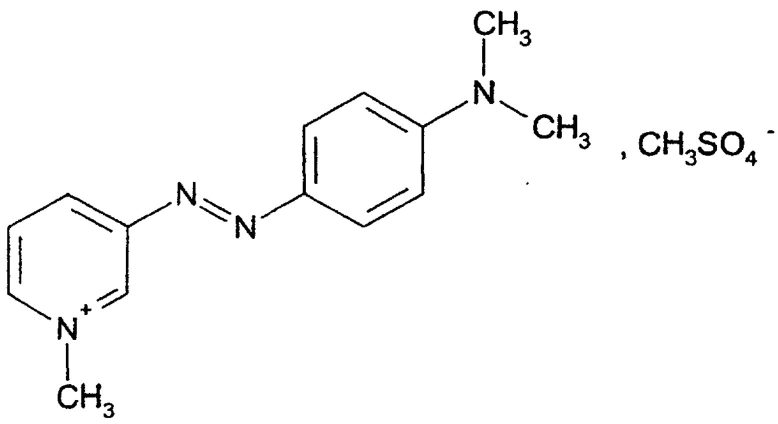

;

- le méthosulfate de bis-(β-hydroxyéthyl)amino-4'-benzène-azo-1' : 3-méthyl-1-pyridinium de formule :

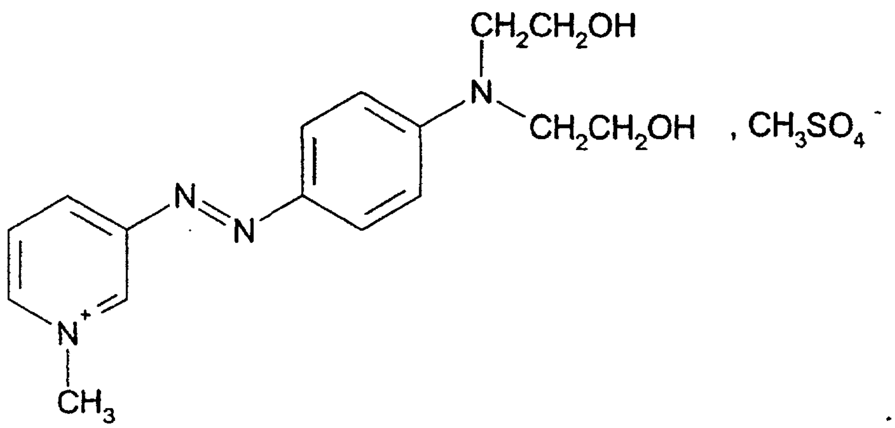

;

- le méthosulfate d'amino-4'-hydroxy-8'-naphtalène-azo-1' : 3-méthyl-1-pyridinium de formule :

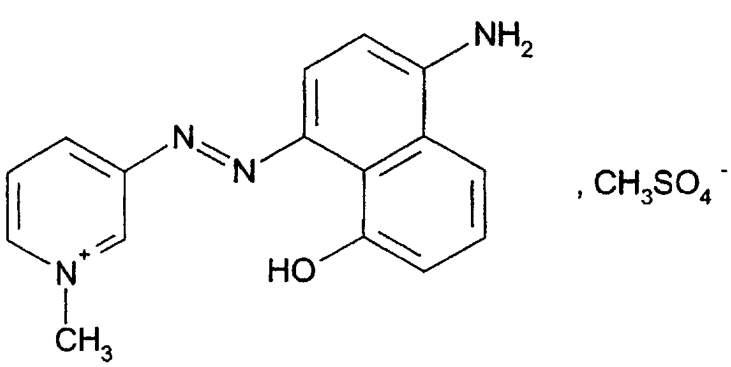

;

- le méthosulfate de diméthylamino-4'-nitro-2'-benzène-azo-1' : 3-méthyl-1-pyridinium de formule :

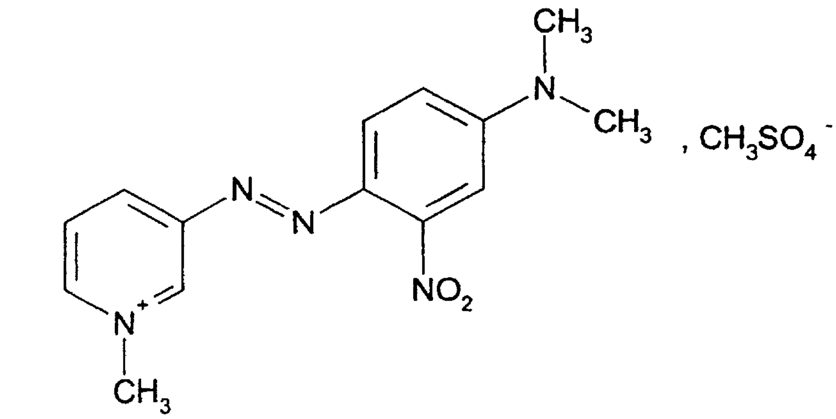

;

- le méthosulfate de diméthylamino-4'-benzène-azo-1' : 3-diméthyl-1,6-pyridinium de formule :

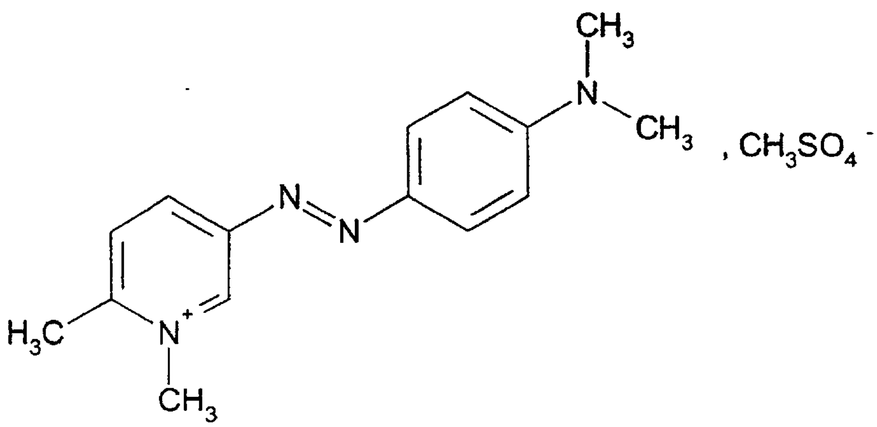

;

- l'amino-4'-benzène-azo-1' : 3-pyridine N-oxyde de formule :

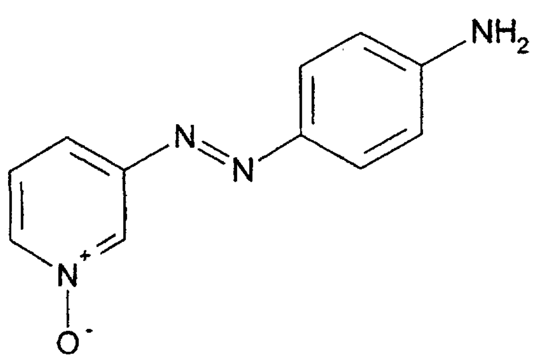

;

- la diméthylamino-4'-benzène-azo-1' : 3-pyridine N-oxyde de formule :

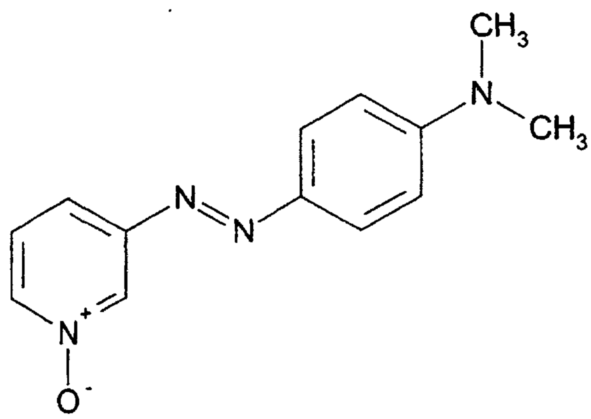

;

- la N,N-bis-(β-hydroxyéthyl)amino-4'-benzène-azo-1' : 3-pyridine N-oxyde de formule :

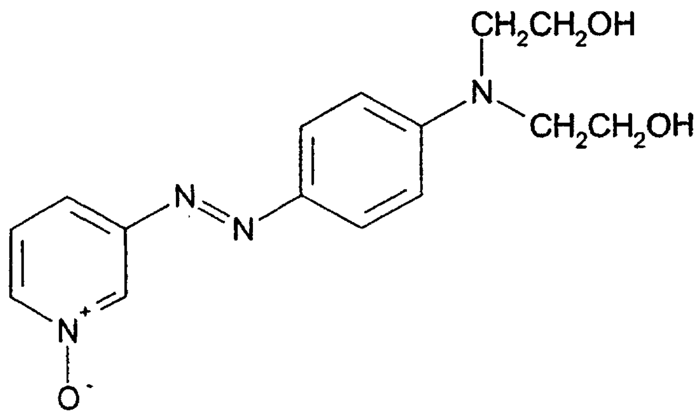

;

- l'éthosulfate de diméthylamino-4'-méthyl-2'-benzène-azo-1' : 3-éthyl-1-pyridinium de formule :

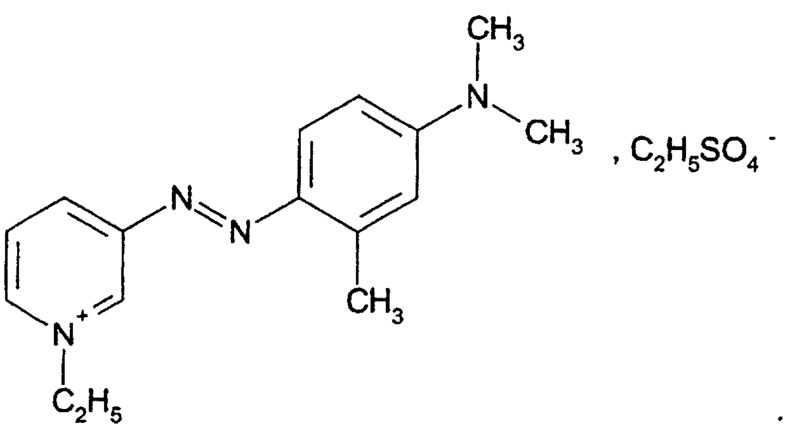

;

- le bromure de diméthylamino-4'-méthyl-2'-benzène-azo-1' : 3-butyl-1-pyridinium de formule :

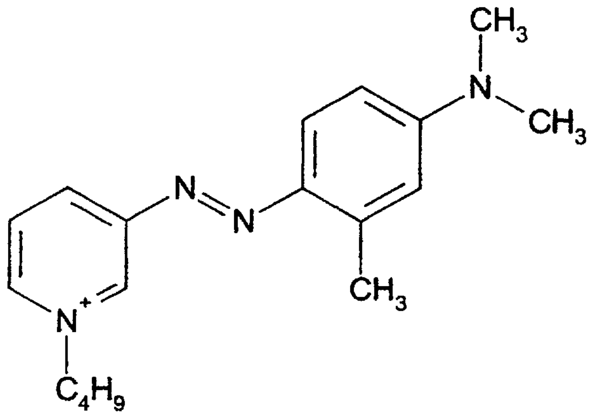

$, Br^-$

;

- le méthosulfate de diméthylamino-4'-chloro-2'-benzène-azo-1': 3-méthyl-1-pyridinium de formule :

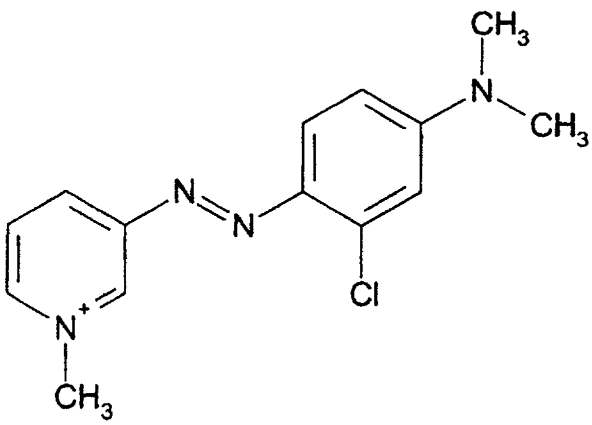

$, CH_3SO_4^-$

;

- le méthosulfate de diamino-2',4'-méthyl-5'-benzène-azo-1': 3-méthyl-1 pyridinium de formule :

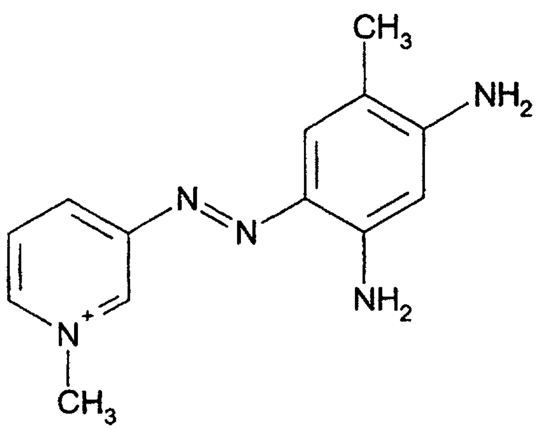

$, CH_3SO_4^-$

;

- le méthosulfate de phénylamino-4'-benzène-azo-1': 3-méthyl-1-pyridinum de formule

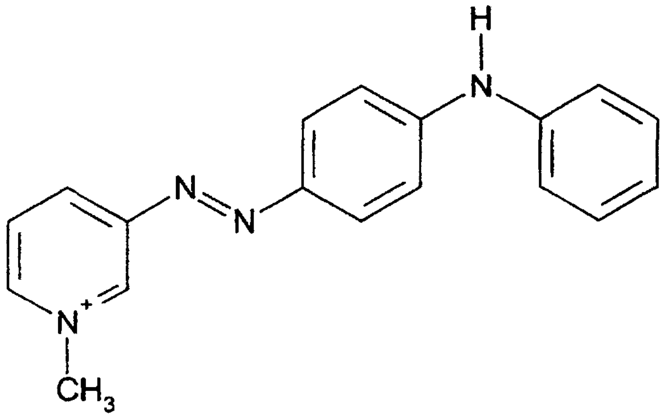

, $CH_3SO_4^-$

;

- l'éthosulfate d'acétylamino-2'-diméthylamino-4'-benzène-azo-1': 3-éthyl-1-pyridinium de formule :

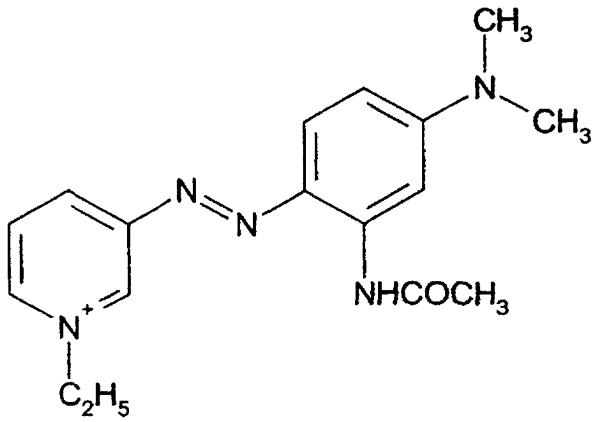

, $C_2H_5SO_4^-$

;

- le méthosulfate de diamino-2',4'-méthoxy-5'-benzène-azo-1' : 3-méthyl pyridinium de formule

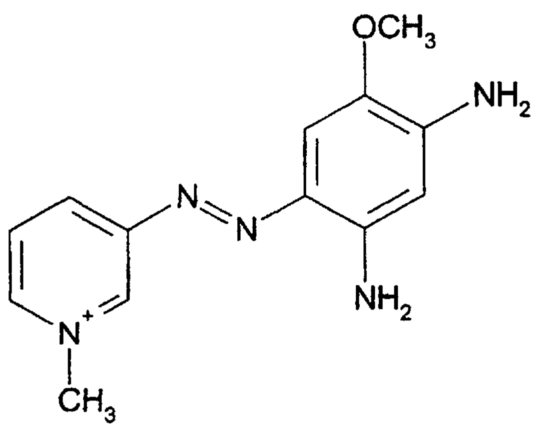

, $CH_3SO_4^-$

;

et

- le méthosulfate d'amino-2'-diméthylamino-4'-benzène-azo-1' : 3-méthyl-1 pyridinium de formule :

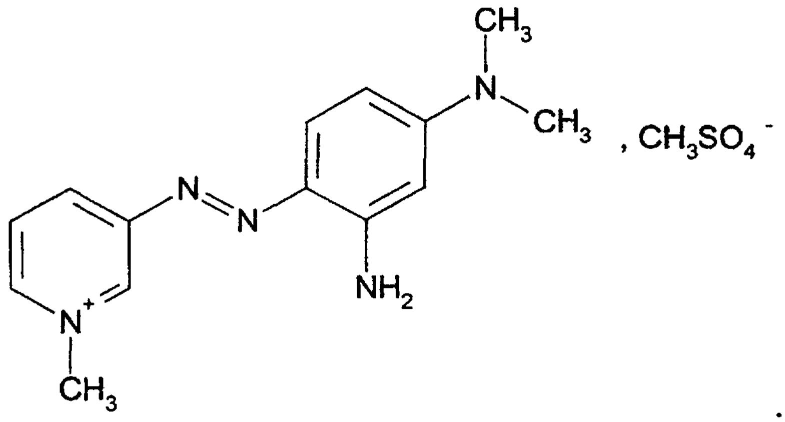

Le ou les dérivés de 3-amino pyridine de formule (I) utilisés selon l'invention, représentent de préférence de 0,001 à 10 % en poids environ du poids total de la composition tinctoriale et encore plus préférentiellement de 0,01 à 5 % en poids environ de ce poids.

Parmi les méta-aminophénols de formule (II) ci-dessus, on peut plus particulièrement citer le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, le 3-amino 2-chloro 6-méthyl phénol, le 3-amino 6-chloro phénol, le 3-(β-aminoéthyl)amino 6-chloro phénol, et leurs sels d'addition avec un acide.

Le ou les dérivés de méta-aminophénol de formule (II) conformes à l'invention représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut en outre contenir un ou plusieurs coupleurs différents des dérivés de méta-aminophénol de formule (II) et/ou un ou plusieurs colorants directs différents des dérivés de 3-amino pyridine de formule (I), notamment pour modifier les nuances ou les enrichir en reflets.

Parmi les coupleurs pouvant être présents à titre additionnel dans la composition tinctoriale conforme à l'invention, on peut notamment citer les méta-phényiènediamines, les métadiphénols, les coupleurs hétérocycliques, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en $C_1$-$C_4$, tels que l'éthanol et l'isopropanol.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (VI) suivante :

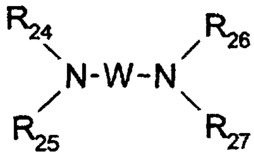 (VI)

dans laquelle W est un reste propylène éventuellement substitué par un groupement hydroxyle ou un radical alkyle en $C_1$-$C_4$ ; $R_{24}$, $R_{25}$, $R_{26}$ et $R_{27}$, identiques ou différents, représentent un atome d'hydrogène, un radical alkyle en $C_1$-$C_4$ ou hydroxyalkyle en $C_1$-$C_4$.

La composition tinctoriale conforme à l'invention peut également renfermer divers adjuvants utilisés classiquement dans les compositions pour la teinture des cheveux.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains.

L'invention a également pour objet un procédé de teinture des fibres kératiniques, et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates, les percarbonates et persulfates, les peracides, les enzymes telles que les oxydo-réductases à 2 électrons, les peroxydases et les laccases. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

## EXEMPLES

EXEMPLES DE TEINTURE COMPARATIFS 1 A 4

On a préparé les compositions tinctoriales suivantes (teneurs en grammes) :

| EXEMPLE | 1 | 2(*) | 3 | 4 (*) |
|---|---|---|---|---|
| Diméthylamino-4'-benzène-azo-1' : 3-pyridine N-oxyde (composé de formule (I)) | 0,5 | 0,5 | - | - |
| Ethosulfate d'acétylamino-2'-diméthylamino-4'-benzène-azo-1' : 3-éthyl-1-pyridinium (composé de formule (I)) | - | - | 0,6 | 0,6 |
| Paraphénylènediamine (base d'oxydation) | 0,324 | 0,324 | 0,324 | 0,324 |
| 5-amino 2-méthyl phénol (coupleur de formule (II)) | 0,369 | - | 0,369 | - |
| Méta-aminophénol (coupleur) | - | 0,327 | - | 0,327 |
| Support de teinture commun | (**) | (**) | (**) | (**) |
| Eau déminéralisée qsp | 100 g | 100 g | 100 g | 100 g |

(*) : Exemple comparatif ne faisant pas partie de l'invention

(**) : Support de teinture commun :
- Alcool oléique polyglycérolé à 2 moles de glycérol 4,0 g
- Alcool oléique polyglycérolé à 4 moles de glycérol à 78 % de matières actives (M.A.) 5,69 g M.A.
- Acide oléique 3,0 g
- Aminé oléique à 2 moles d'oxyde d'éthylène vendue sous la dénomination commerciale ETHOMEEN O12 ® par la société AKZO 7,0 g
- Laurylamino succinamate de diéthylaminopropyle, sel de sodium à 55 % de M.A. 3,0 g MA.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium à en solution aqueuse à 35 % de M.A. 0,455 g M.A.
- Acétate d'ammonium 0,8 g
- Antioxydant, séquestrant q.s.
- Parfum, conservateur q.s.
- Ammoniaque à 20 % de $NH_3$ 10,0 g

Au moment de l'emploi, on a mélangé chacune des compositions tinctoriales décrites ci-dessus avec une quantité pondérale équivalente de peroxyde d'hydrogène à 20 volumes (6% en poids) présentant un pH d'environ 3.

Chaque mélange résultant présentait un pH d'environ 10 ± 0,2 et a été appliqué pendant 30 minutes sur des mèches de cheveux gris à 90 % de blancs permanentés.

Les cheveux ont ensuite été rincés à l'eau, lavés avec un shampooing standard, rincés à nouveau puis séchés.

La couleur des mèches a été évaluée avant et après la teinture, dans le système Munsell, au moyen d'un spectrophotomètre CM 2002 MINOLTA ®.

Selon la notation Munsell, une couleur est définie par la formule :

$$HV/C$$

dans laquelle les trois paramètres désignent respectivement la "Hue" ou nuance (H), la "Value" ou intensité (V) et le "Chroma" ou saturation (C), la barre oblique étant simplement une convention et ne désignant pas un ratio.

La montée de la coloration ΔE peut être calculée en appliquant l'équation de Nickerson :

$$\Delta E = 0.4C_0dH + 6dV + 3dC$$

telle que décrite par exemple dans "Journal of the Optical Society of America", vol.34, N°. 9, Sept 1944, pages 550-570.

Dans cette équation, ΔE représente la différence de couleur entre deux mèches, (dans le cas présent la montée de la coloration), dH, dV, et dC représente la variation en valeur absolue des trois paramètres H, V, et C, $C_o$ représentant la saturation de la mèche par rapport à laquelle on veut évaluer la différence de couleur.

Plus la valeur de ΔE est importante, plus la différence de couleur entre les deux mèches est importante, et dans le cas présent, plus la montée de la coloration, (ou puissance de la coloration), est importante.

Les résultats figurent dans le tableau ci-après :

| Exemple | Couleur de la mèche avant teinture | Couleur de la mèche après teinture | Montée de la coloration | | | |
|---|---|---|---|---|---|---|
| | | | dH | dV | dC | ΔE |
| **1** | 3,3 Y 5,8 / 1,6 | 6,5 R 2,7 / 3,8 | 16,8 | 3,1 | 2,2 | **36,0** |
| **2 (*)** | 3,3 Y 5,8 / 1,6 | 1,2 YR 2,4 / 2,1 | 12,1 | 3,4 | 0,5 | **29,6** |
| **3** | 3,3 Y 5,8 / 1,6 | 5,1 R 2,5 / 3,0 | 18,2 | 3,3 | 1,4 | **35,6** |
| **4 (*)** | 3,3 Y 5,8 / 1,6 | 8,7 R 2,2 / 1,5 | 14,6 | 3,6 | 0,1 | **31,2** |

(*) exemple comparatif ne faisant pas partie de l'invention

On constate que les compositions tinctoriales des exemples 1 et 3 conformes à l'invention, c'est à dire contenant l'association d'un colorant direct de formule (I), d'une base d'oxydation et d'un coupleur de formule (II) conduisent à des colorations plus puissantes que les compositions tinctoriales des exemples 2 et 4 ne faisant pas partie de l'invention dans la mesure où elles contiennent un coupleur de type méta-aminophénol non substitué et telles que décrites par exemple dans la demande de brevet FR-A-2 285 851.

## Revendications

1. Composition pour la teinture d'oxydation des fibres kératiniques et en particulier des fibres kératiniques humaines telles que les cheveux, **caractérisée par le fait qu'**elle comprend, dans un milieu approprié pour la teinture :

- au moins une base d'oxydation,
- à titre de colorant direct, au moins un dérivé de 3-amino pyridine choisi parmi les composés de formule (I) suivante :

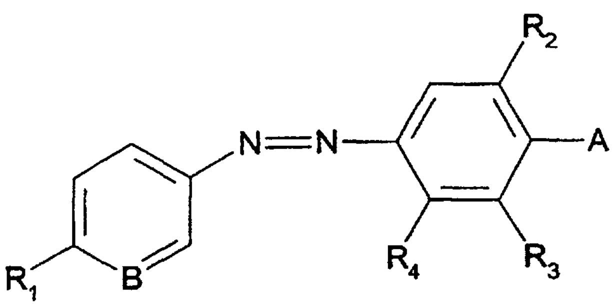

(I)

dans laquelle :

- B représente un groupement de formules (Ia) ou (Ib) suivantes :

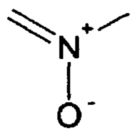

(Ia)

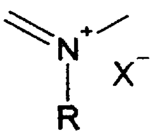

(Ib)

- R représente un radical alkyle en $C_1$-$C_4$ ;
- $R_1$ représente un atome d'hydrogène ou d'halogène tel que le chlore, le brome ou le fluor, un radical alkyle en $C_1$-$C_4$ ou alcoxy en $C_1$-$C_4$ ;

- $R_2$ représente un atome d'hydrogène, un radical alkyle en $C_1$-$C_4$ ou alcoxy en $C_1$-$C_4$ ;
- $R_4$ représente un atome d'hydrogène ou d'halogène tel que le chlore, le brome ou le fluor, un radical alkyle en $C_1$-$C_4$, nitro, amino ou acyl($C_1$-$C_4$)amino ;
- $R_3$ représente un atome d'hydrogène ou bien $R_4$ et $R_3$ forment un cycle insaturé à 6 chaînons portant un substituant hydroxyle chélaté avec un des atomes d'azote de la double liaison azoïque :
- A représente un reste -$NR_5R_6$ dans lequel $R_5$ représente un atome d'hydrogène, un radical alkyle en $C_1$-$C_4$, monohydroxyalkyle en $C_1$-$C_4$ ou polyhydroxyalkyle en $C_2$-$C_4$ ; $R_6$ représente un atome d'hydrogène, un radical alkyle en $C_1$-$C_4$, monohydroxyalkyle en $C_1$-$C_4$ ou polyhydroxyalkyle en $C_2$-$C_4$, un cycle phényle ou un radical -$CH_2$-$SO_3Na$ ;
- $X^-$ représente un anion monovalent ou divalent et est de préférence choisi parmi un atome d'halogène tel que le chlore, le brome, le fluor ou l'iode, un hydroxyde, un hydrogènesulfate, ou un alkyl($C_1$-$C_6$)sulfate tel que par exemple un méthylsulfate ou un éthylsulfate, et
- au moins un coupleur choisi parmi les dérivés de méta-aminophénol de formule (II) suivante et leurs sels d'addition avec un acide :

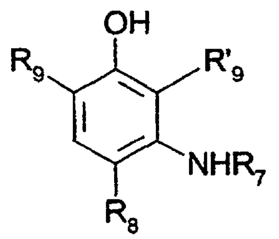
(II)

dans laquelle :

- $R_7$ représente un atome d'hydrogène, un radical alkyle en $C_1$-$C_4$, monohydroxyalkyle en $C_1$-$C_4$, polyhydroxyalkyle en $C_2$-$C_4$ ou monoaminoalkyle en $C_1$-$C_4$ ;
- $R_8$ représente un atome d'hydrogène, un radical alkyle en $C_1$-$C_4$, alcoxy en $C_1$-$C_4$ ou un atome d'halogène choisi parmi le chlore, le brome ou le fluor,
- $R_9$ et $R'_9$, identiques ou différents, représentent un atome d'hydrogène ou d'halogène, un radical alkyle en $C_1$-$C_4$, alcoxy en $C_1$-$C_4$, monohydroxyalkyle en $C_1$-$C_4$, polyhydroxyalkyle en $C_2$-$C_4$, monohydroxyalcoxy en $C_1$-$C_4$ -ou polyhydroxyalcoxy en $C_2$-$C_4$ ;

étant entendu qu'au moins un des radicaux $R_7$, $R_8$, $R_9$ et $R'_9$ est différent d'un atome d'hydrogène.

2. Composition selon la revendication 1, **caractérisée par le fait que** la ou les bases d'oxydation sont choisies parmi les paraphénylènediamines, les bases doubles, les para-aminophénols, les ortho aminophénols et les bases d'oxydation hétérocycliques.

3. Composition selon la revendication 2, **caractérisée par le fait que** les paraphénylènediamines sont choisies parmi les composés de formule (III) suivante et leurs sels d'addition avec un acide :

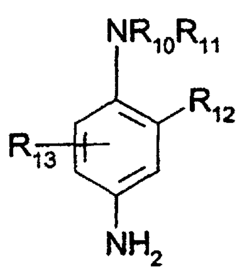
(III)

dans laquelle :

- $R_{10}$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phé-

nyle ou 4'-aminophényle ;

- $R_{11}$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_{12}$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$, hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,
- $R_{13}$ représente un atome d'hydrogène, d'halogène ou un radical alkyle en $C_1$-$C_4$.

**4.** Composition selon la revendication 3, **caractérisée par le fait que** les paraphénylènediamine de formule (III) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl) amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

**5.** Composition selon la revendication 2, **caractérisée par le fait que** les bases doubles sont choisies parmi les composés de formule (IV) suivante, et leurs sels d'addition avec un acide :

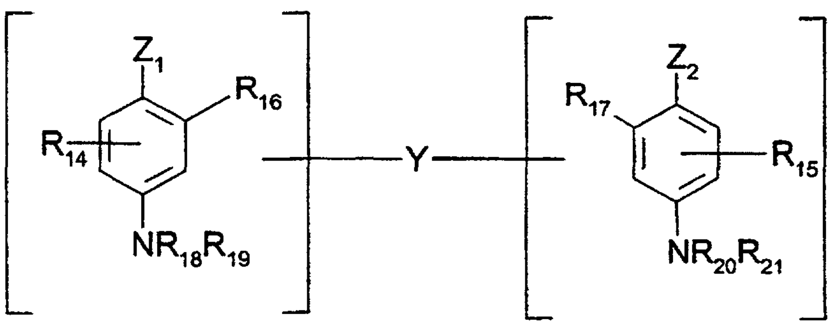

(II)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_{14}$ et $R_{15}$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_{16}$, $R_{17}$, $R_{18}$, $R_{19}$ $R_{20}$ et $R_{21}$, identiques ou différents, représentent un atome d'hydrogène, un bras de liaison Y ou un radical alkyle en $C_1$-$C_4$ ;

étant entendu que les composés de formule (IV) ne comportent qu'un seul bras de liaison Y par molécule.

**6.** Composition selon la revendication 5, **caractérisée par le fait que** les bases doubles de formule (IV) sont choisies parmi le N,N'-bis-(β-hydroxyéthyl) N,N'-bis-(4'-aminophényl) 1,3-diamino propanol, la N,N'-bis-(β-hydroxyéthyl) N, N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène

diamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

**7.** Composition selon la revendication 2, **caractérisée par le fait que** para-aminophénols sont choisis parmi les composés de formule (V) suivante, et leurs sels d'addition avec un acide :

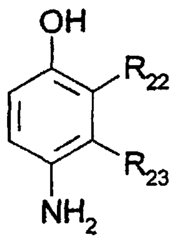

(V)

dans laquelle :

- $R_{22}$ représente un atome d'hydrogène ou d'halogène, un radical alkyle en $C_1$-$C_4$, monohydroxyalkyle en $C_1$-$C_4$, alcoxy($C_1$-$C_4$)alkyle($C_1$-$C_4$), aminoalkyle en $C_1$-$C_4$ ou hydroxyalkyl($C_1$-$C_4$)aminoalkyle en $C_1$-$C_4$,
- $R_{23}$ représente un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),

étant entendu qu'au moins un des radicaux $R_{22}$ et $R_{23}$ représente un atome d'hydrogène.

**8.** Composition selon la revendication 7, **caractérisée par le fait que** les para-aminophénols de formule (V) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

**9.** Composition selon la revendication 2, **caractérisée par le fait que** les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

**10.** Composition selon la revendication 2, **caractérisée par le fait que** les bases d'oxydation hétérocycliques sont choisies parmi les dérivés pyridiniques, les dérivés pyrimidiniques, les dérivés pyrazoliques, et leurs sels d'addition avec un acide.

**11.** Composition selon l'une quelconque des revendications précédentes, **caractérisée par le fait que** la ou les bases d'oxydation représentent de 0,0005 à 12 % en poids du poids total de la composition tinctoriale.

**12.** Composition selon la revendication 11, **caractérisée par le fait que** la ou les bases d'oxydation représentent de 0,005 à 6 % en poids du poids total de la composition tinctoriale.

**13.** Composition selon l'une quelconque des revendications précédentes, **caractérisée par le fait que** le ou les dérivés de 3-amino pyridine de formule (I) sont choisis parmi :

- le méthosulfate de diméthylamino-4' benzène azo-1' : 3-méthyl-1-pyridinium de formule :

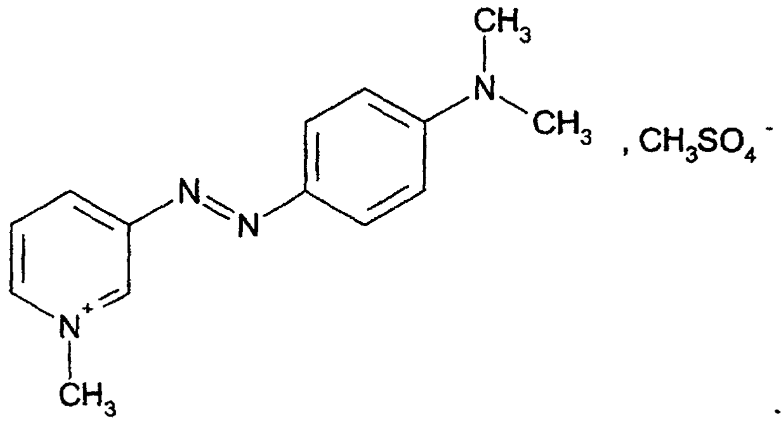

;

- le méthosulfate de bis-(β-hydroxyéthyl)amino-4'-benzène-azo-1' : 3-méthyl-1-pyridinium de formule :

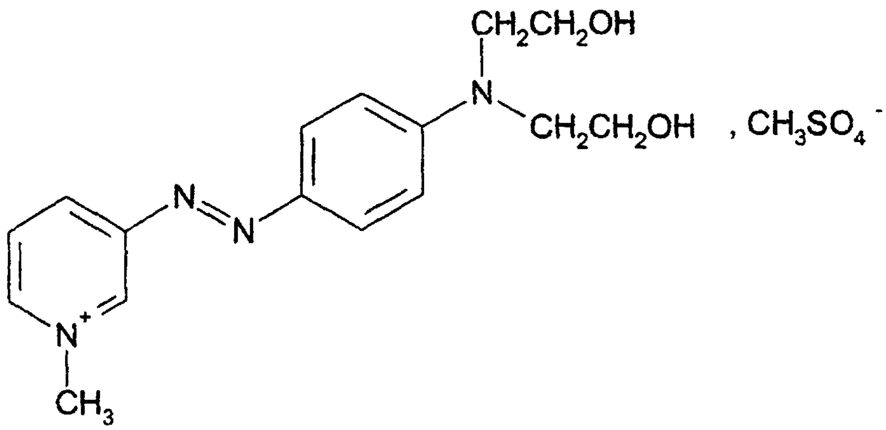

;

- le méthosulfate d'amino-4'-hydroxy-8'-naphtalène-azo-1': 3-méthyl-1-pyridinium de formule :

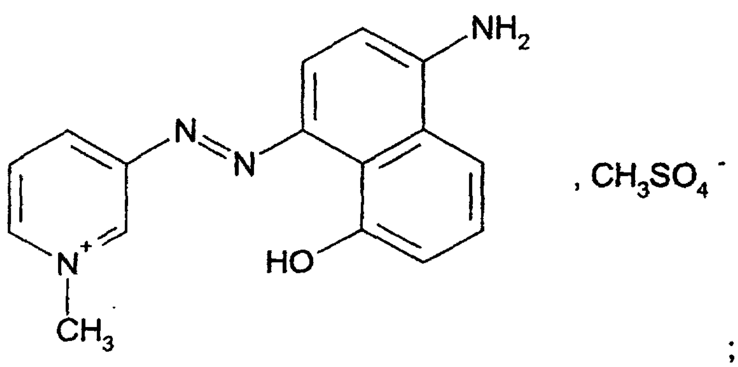

;

- le méthosulfate de diméthylamino-4'-nitro-2'-benzène-azo-1': 3-méthyl-1-pyridinium de formule :

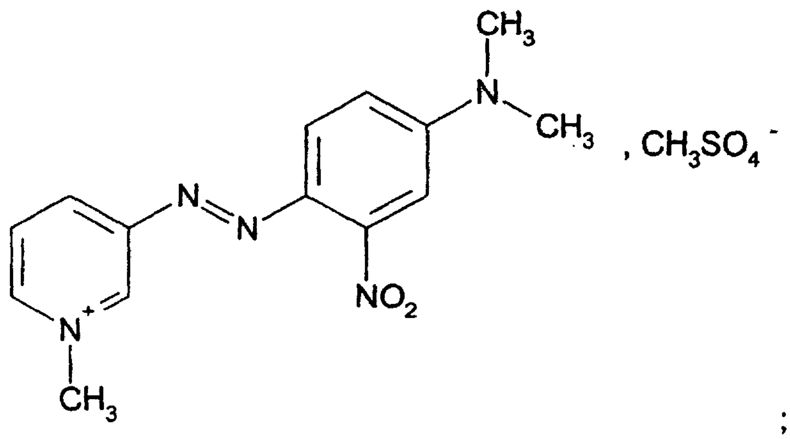

;

- le méthosulfate de diméthylamino-4'-benzène-azo-1' : 3-diméthyl-1,6-pyridinium de formule :

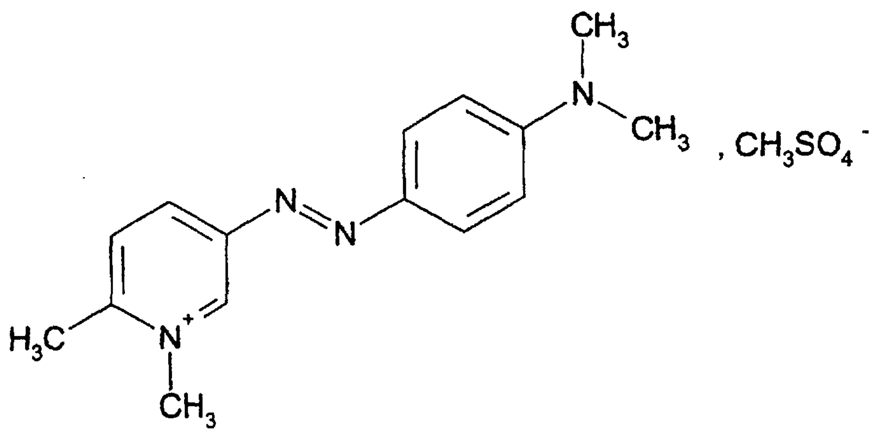

;

- l'amino-4'-benzène-azo-1' : 3-pyridine N-oxyde de formule :

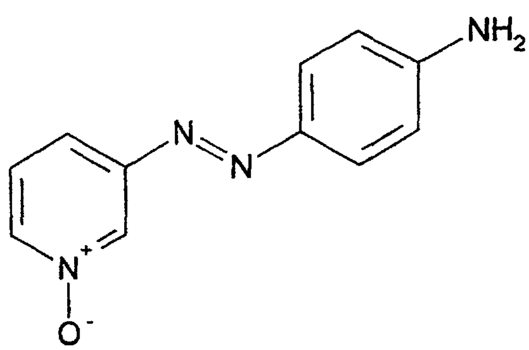

;

- la diméthylamino-4'-benzène-azo-1': 3-pyridine N-oxyde de formule :

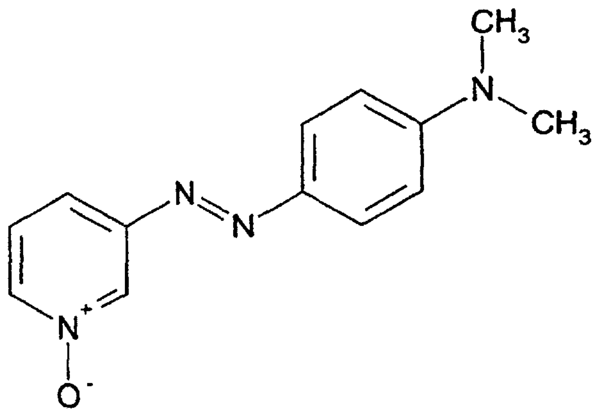

;

- la N,N-bis-(β-hydroxyéthyl)amino-4'-benzène-azo-1': 3-pyridine N-oxyde de formule :

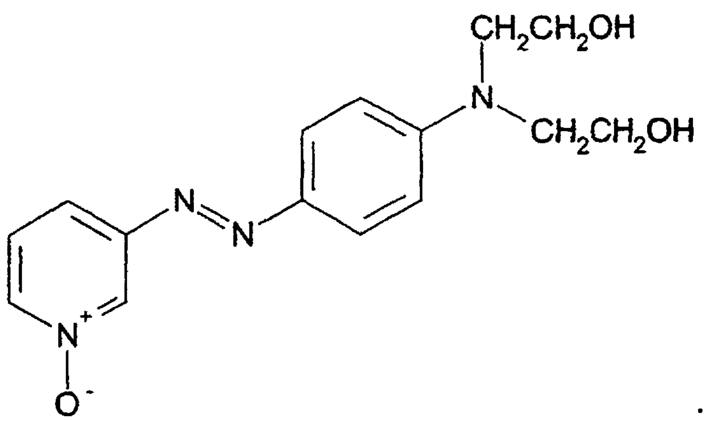

;

- l'éthosulfate de diméthylamino-4'-méthyl-2'-benzène-azo-1' : 3-éthyl-1-pyridinium de formule :

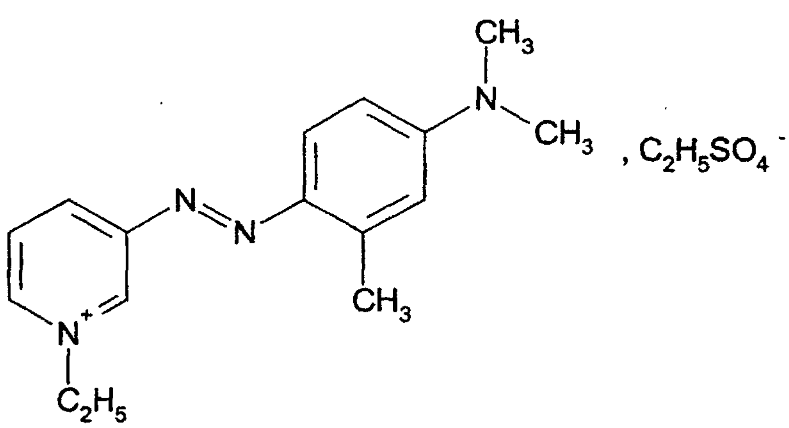

;

- le bromure de diméthylamino-4'-méthyl-2'-benzène-azo-1' : 3-butyl-1-pyridinium de formule :

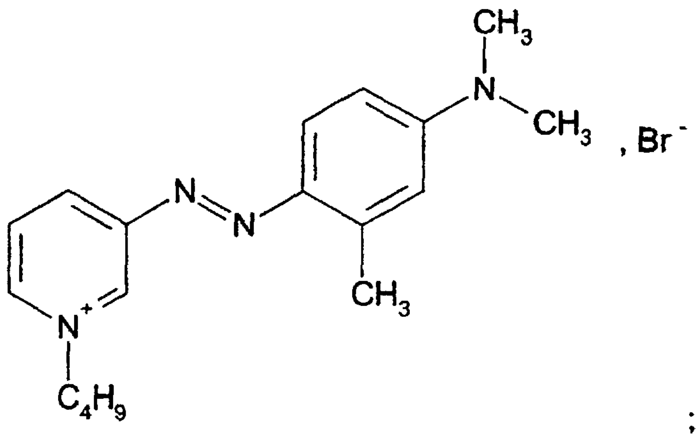

;

- le méthosulfate de diméthylamino-4'-chloro-2'-benzène-azo-1' : 3-méthyl-1-pyridinium de formule :

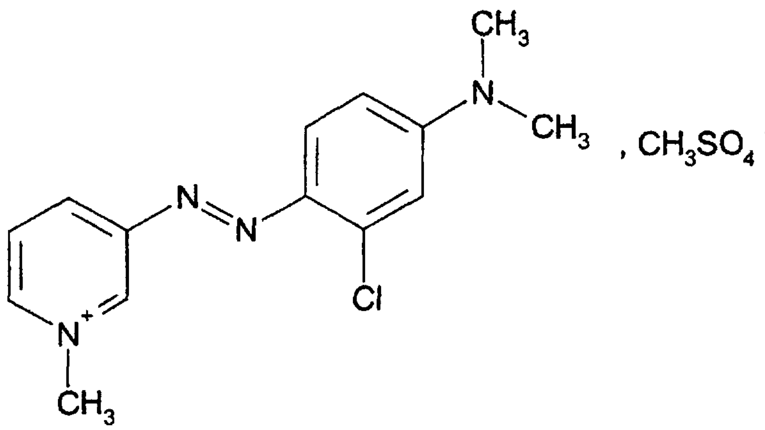

;

- le méthosulfate de diamino-2',4'-méthyl-5'-benzène-azo-1' : 3-méthyl-1 pyridinium de formule :

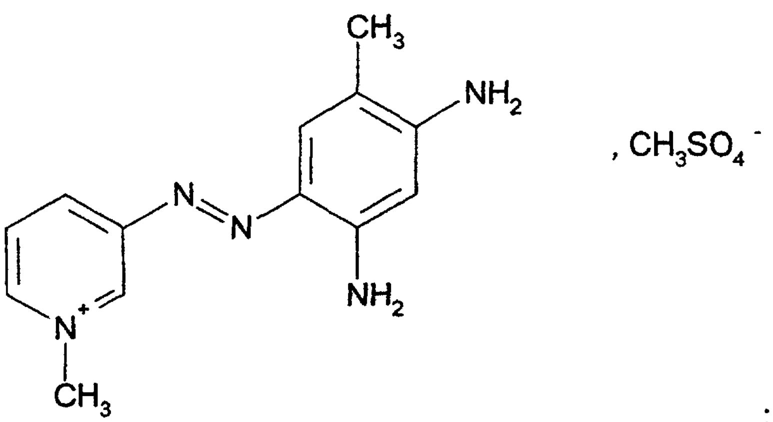

;

- le méthosulfate de phénylamino-4'-benzène-azo-1' : 3-méthyl-1-pyridinum de formule

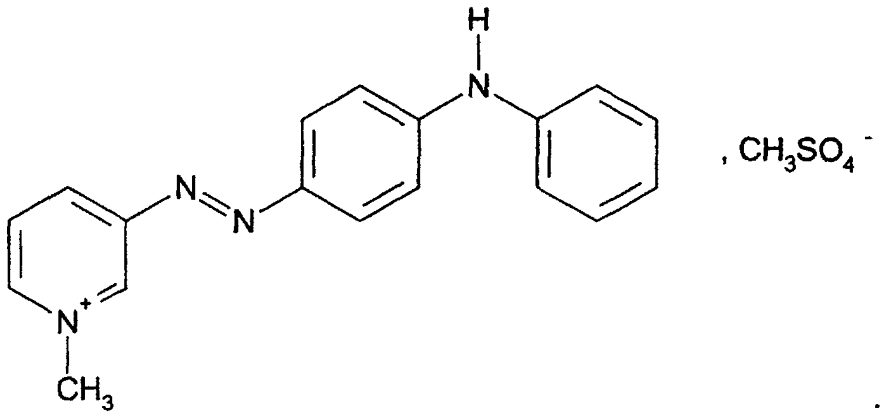

;

- l'éthosulfate d'acétylamino-2'-diméthylamino-4'-benzène-azo-1': 3-éthyl-1-pyridinium de formule :

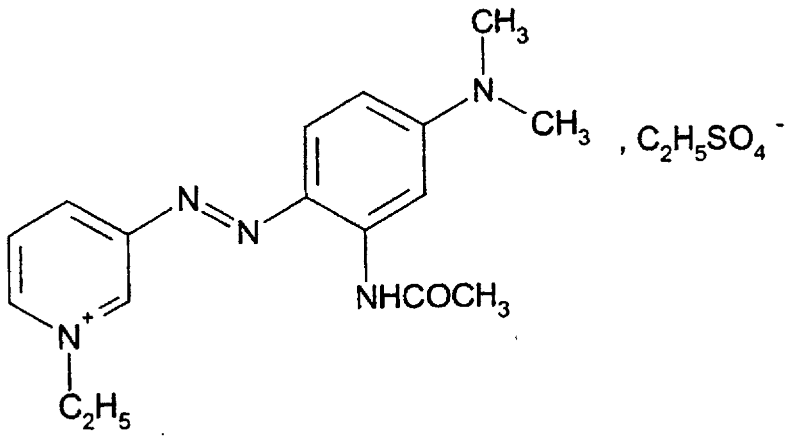

;

- le méthosulfate de diamino-2',4'-méthoxy-5'-benzène-azo-1' : 3-méthyl pyridinium de formule

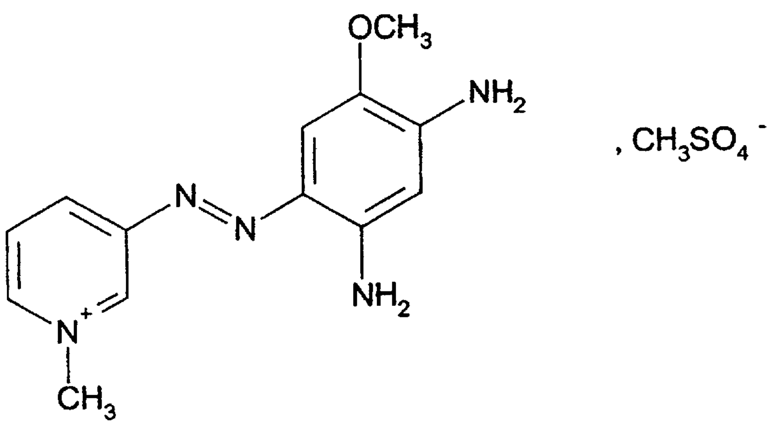

;

et

- le méthosulfate d'amino-2'-diméthylamino-4'-benzène-azo-1' : 3-méthyl-1 pyridinium de formule :

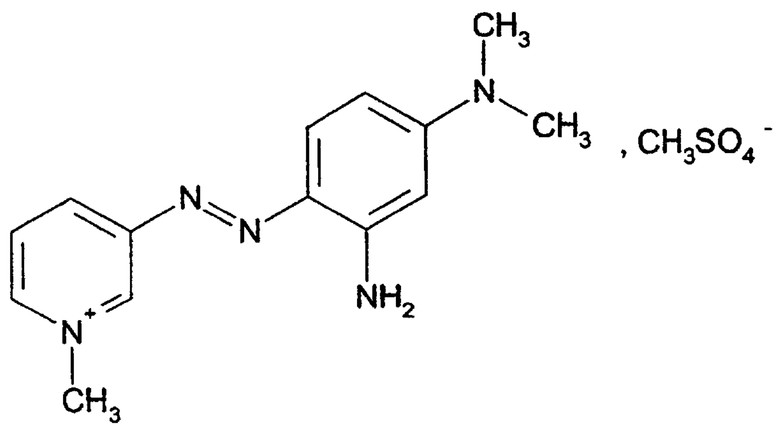

**14.** Composition selon l'une quelconque des revendications précédentes, **caractérisée par le fait que** le ou les dérivés de 3-amino pyridine de formule (I) représentent de 0,001 à 10 % en poids du poids total de la composition tinctoriale.

**15.** Composition selon la revendication 14, **caractérisée par le fait que** le ou les dérivés de 3-amino pyridine de formule (I) représentent de 0,01 à 5 % en poids du poids total de la composition tinctoriale.

**16.** Composition selon l'une quelconque des revendications précédentes, **caractérisée par le fait que** les dérivés de méta-aminophénol de formule (II) sont choisis parmi le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl) amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, le 3-amino 2-chloro 6-méthyl phénol, le 3-amino 6-chloro phénol, le 3-(β-aminoéthyl)amino 6-chloro phénol, et leurs sels d'addition avec un acide.

**17.** Composition selon l'une quelconque des revendications précédentes, **caractérisée par le fait que** le ou les dérivés de méta-aminophénol de formule (II) représentent de 0,0001 à 10 % en poids du poids total de la composition tinctoriale.

**18.** Composition selon la revendication 17, **caractérisée par le fait que** le ou les dérivés de méta-aminophénol de formule (II) représentent de 0,005 à 5 % en poids du poids total de la composition tinctoriale.

**19.** Composition selon l'une quelconque des revendications précédentes, **caractérisée par le fait qu'**elle renferme un ou plusieurs coupleurs différents des dérivés de méta-aminophénol de formule (II) tels que définis à la revendication 1 et/ou un ou plusieurs colorants directs différents des dérivés de 3-amino pyridine de formule (I) tels que définis à la revendication 1.

**20.** Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

**21.** Composition selon l'une quelconque des revendications précédentes, **caractérisée par le fait que** le milieu approprié pour la teinture est constitué par de l'eau ou par un mélange d'eau et d'au moins un solvant organique.

**22.** Composition selon l'une quelconque des revendications précédentes, **caractérisée par le fait qu'**elle présente un pH compris 3 et 12.

**23.** Procédé de teinture des fibres kératiniques et en particulier des fibres kératiniques humaines telles que les cheveux, **caractérisé par le fait qu'**on applique sur lesdites fibres au moins une composition tinctoriale telle que définie à l'une quelconque des revendications 1 à 22, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

**24.** Procédé selon la revendication 23, **caractérisé par le fait que** l'agent oxydant présent dans la composition oxydante est choisi parmi le peroxyde d'hydrogène, le peroxyde d'urée, les bromates de métaux alcalins, les persels tels que les perborates, les percarbonates et persulfates, les peracides, et les enzymes.

**25.** Dispositif à plusieurs compartiments, ou "kit" de teinture à plusieurs compartiments, dont un premier compartiment renferme une composition tinctoriale telle que définie à l'une quelconque des revendications 1 à 22 et un second compartiment renferme une composition oxydante.

**Patentansprüche**

**1.** Zusammensetzung zum oxidativen Färben von Keratinfasern und insbesondere menschlichen Keratinfasern, wie dem Haar, **dadurch gekennzeichnet, dass** sie in einem zum Färben geeigneten Medium enthält:

- mindestens eine Oxidationsbase,
- als Direktfarbstoff mindestens ein 3-Amino-pyridinderivat, das unter den Verbindungen der folgenden Formel (I) ausgewählt ist:

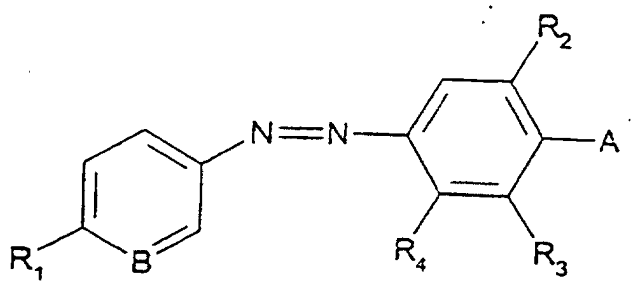

(I)

worin bedeuten:

- B eine Gruppe der folgenden Formeln (Ia) oder (Ib):

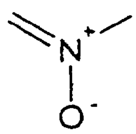

(Ia)

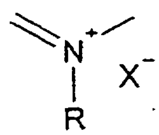

(Ib)

- R eine $C_{1-4}$-Alkylgruppe;
- $R_1$ ein Wasserstoffatom, ein Halogenatom, wie Chlor, Brom oder Fluor, eine $C_{1-4}$-Alkylgruppe oder eine $C_{1-4}$-Alkoxygruppe;
- $R_2$ ein Wasserstoffatom, eine $C_{1-4}$-Alkylgruppe oder eine $C_{1-4}$-Alkoxygruppe;
- $R_4$ ein Wasserstoffatom, ein Halogenatom, wie Chlor, Brom oder Fluor, eine $C_{1-4}$-Alkylgruppe, Nitro, Amino oder eine $C_{1-4}$-Acylaminogruppe;
- $R_3$ ein Wasserstoffatom oder die Gruppen $R_4$ und $R_3$ bilden einen ungesättigen 6-gliedrigen Ring, der einen Hydroxysubstituenten trägt, der ein Chelat mit den beiden Stickstoffatomen der Azo-Doppelbindung bildet;
- A eine Gruppe $-NR_5R_6$, wobei $R_5$ Wasserstoff, $C_{1-4}$-Alkyl, $C_{1-4}$-Monohydroxyalkyl oder $C_{2-4}$-Polyhydroxyalkyl und $R_6$ Wasserstoff, $C_{1-4}$-Alkyl, $C_{1-6}$-Monohydroxyalkyl, $C_{2-6}$-Polyhydroxyalkyl, Phenyl oder $-CH_2-SO_3Na$ bedeutet;
- die Gruppe $X^-$ ein einwertiges oder zweiwertiges Anion, wobei sie vorzugsweise unter den Halogenatomen, wie Chlor, Brom, Fluor oder Iod, Hydroxid, Hydrogensulfat oder Alkyl($C_{1-6}$)sulfat, beispielsweise Methyl- oder Ethylsulfat, ausgewählt ist; und

mindestens einen Kuppler, der unter den m-Aminophenolen der folgenden Formel (II) und deren Additionssalzen mit einer Säure ausgewählt ist:

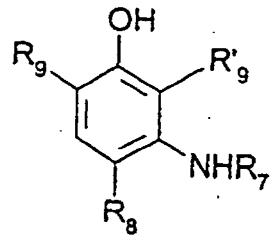

(II),

worin bedeuten:

- $R_7$ Wasserstoff, $C_{1-4}$-Alkyl, $C_{1-4}$-Monohydroxyalkyl, $C_{2-4}$-Polyhydroxyalkyl oder $C_{1-4}$-Monoaminoalkyl;
- $R_8$ Wasserstoff, $C_{1-4}$-Alkyl, $C_{1-4}$-Alkoxy oder ein Halogenatom, das unter Chlor, Brom oder Fluor ausgewählt ist,
- $R_9$ und $R_{9'}$, die gleich oder verschieden sind, Wasserstoff, Halogen, $C_{1-4}$-Alkyl, $C_{1-4}$-Alkoxy, $C_{1-4}$-Monohydroxyalkyl, $C_{2-4}$-Polyhydroxyalkyl, $C_{1-4}$-Monohydroxyalkoxy oder $C_{2-4}$-Polyhydroxyalkoxy;

mit der Maßgabe, dass mindestens eine der Gruppen $R_7$, $R_8$, $R_9$ und $R_{9'}$ von Wasserstoff verschieden ist.

**2.** Zusammensetzung nach Anspruch 1, **dadurch gekennzeichnet, dass** die Oxidationsbase(n) unter den p-Phenylendiaminen, Doppelbasen, p-Aminophenolen, o-Aminophenolen und den heterocyclischen Oxidationsbasen ausgewählt sind.

**3.** Zusammensetzung nach Anspruch 2, **dadurch gekennzeichnet, dass** die p-Phenylendiamine unter den Verbindungen der folgenden Formel (III) und deren Additionssalzen mit einer Säure ausgewählt sind:

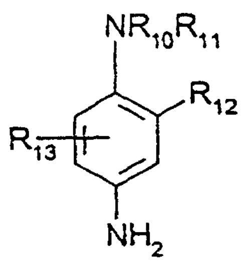

(III),

worin bedeuten:

- $R_{10}$ Wasserstoff, $C_{1-4}$-Alkyl, $C_{1-4}$-Monohydroxyalkyl, $C_{2-4}$-Polyhydroxyalkyl, $C_{1-4}$-Alkoxy-$C_{1-4}$-alkyl, eine mit einer stickstoffhaltigen Gruppe substituierte $C_{1-4}$-Alkylgruppe, Phenyl oder 4'-Aminophenyl,
- $R_{11}$ Wasserstoff, $C_{1-4}$-Alkyl, $C_{1-4}$-Monohydroxyalkyl, $C_{2-4}$-Polyhydroxyalkyl, $C_{1-4}$-Alkoxy-$C_{1-4}$-alkyl oder eine mit einer stickstoffhaltigen Gruppe substituierte $C_{1-4}$-Alkylgruppe,
- $R_{12}$ Wasserstoff, Halogen, wie Chlor, Brom, Iod oder Fluor, $C_{1-4}$-Alkyl, $C_{1-4}$-Monohydroxyalkyl, $C_{1-4}$-Hydroxyalkoxy, $C_{1-4}$-Acetylaminoalkoxy, $C_{1-4}$-Mesylaminoalkoxy oder $C_{1-4}$-Carbamoylaminoalkoxy, und
- $R_{13}$ Wasserstoff, Halogen oder $C_{1-4}$-Alkyl.

**4.** Zusammensetzung nach Anspruch 3, **dadurch gekennzeichnet, dass** die p-Phenylendiamine der Formel (III) unter p-Phenylendiamin, p-Toluylendiamin, 2-Chlor-p-phenylen-diamin, 2,3-Dimethyl-p-phenylendiamin, 2,6-Dimethyl-p-phenylen-diamin, 2,6-Diethyl-pphenylendiamin, 2,5-Dimethyl-p-phenylendiamin, N,N-Dimethyl-pphenylendiamin, N,N-Diethyl-p-phenylendiamin, N,N-Dipropyl-pphenylendiamin, 4-Amino-N,N-diethyl-3-methyl-anilin, N,N-Bis(β-hydroxyethyl)-p-phenylendiamin, 4-N,N-Bis(β-hydroxyethyl)amino-2-methyl-anilin, 4-N,N-Bis(β-hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-pphenylendiamin, N-(β-Methoxyethyl)-p-phenylendiamin und deren Additionssalzen mit einer Säure ausgewählt sind.

5. Zusammensetzung nach Anspruch 2, **dadurch gekennzeichnet, dass** die Doppelbasen unter den Verbindungen der Formel (IV) und deren Additionssalzen mit einer Säure ausgewählt sind:

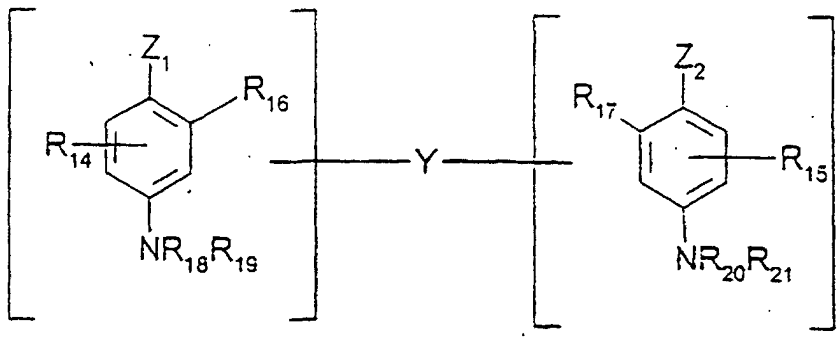

(IV),

worin bedeuten:

- $Z_1$ und $Z_2$, die identisch oder voneinander verschieden sind, Hydroxy oder eine $NH_2$-Gruppe, die mit $C_{1-4}$-Alkyl oder einer Verbindungsgruppe Y substituiert sein kann,
- die Verbindungsgruppe Y eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden kann und gegebenenfalls mit einer oder mehreren Hydroxy- oder $C_{1-6}$-Alkoxygruppen substituiert sein kann,
- $R_{14}$ und $R_{15}$ Wasserstoff, Halogen, $C_{1-4}$-Alkyl, $C_{1-4}$-Monohydroxyalkyl, $C_{2-4}$-Polyhydroxyalkyl, $C_{1-4}$-Aminoalkyl oder eine Verbindungsgruppe Y,
- $R_{16}$, $R_{17}$, $R_{18}$, $R_{19}$, $R_{20}$ und $R_{21}$, die identisch oder voneinander verschieden sind, Wasserstoff, eine Verbindungsgruppe Y oder $C_{1-4}$-Alkyl,

mit der Maßgabe, dass die Verbindungen der Formel (IV) nur eine Verbindungsgruppe Y pro Molekül aufweisen.

6. Zusammensetzung nach Anspruch 5, **dadurch gekennzeichnet, dass** die Doppelbasen der Formel (IV) unter N, N'-Bis(β-hydroxyethyl)-N,N'bis(4'-aminophenyl)-1,3-diamino-propanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis (4-aminophenyl)-tetramethylendiamin, N,N'-Bis(4-methylaminophenyl)-tetramethylendiamin, N,N'-Bis(ethyl)-N, N'-bis-(4'-amino, 3'methylphenyl)-ethylendiamin, 1,8-Bis(2,5-diaminophenoxy)-3,5-dioxaoctan und deren Additionssalzen mit einer Säure ausgewählt sind.

7. Zusammensetzung nach Anspruch 2, **dadurch gekennzeichnet, dass** die p-Aminophenole unter den Verbindungen der Formel (V) und deren Additionssalzen mit einer Säure ausgewählt sind:

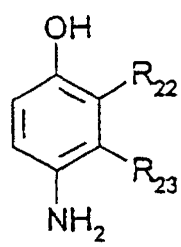

(V),

worin bedeuten:

- $R_{22}$ Wasserstoff, Halogen, $C_{1-4}$-Alkyl, $C_{1-4}$-Monohydroxyalkyl, $C_{1-4}$-Alkoxy-$C_{1-4}$-alkyl, $C_{1-4}$-Aminoalkyl oder $C_{1-4}$-Hydroxyalkyl-$C_{1-4}$aminoalkyl,

- $R_{23}$ Wasserstoff, Halogen, $C_{1-4}$-Alkyl, $C_{1-4}$-Monohydroxyalkyl, $C_{2-4}$-Polyhydroxyalkyl, $C_{1-4}$-Aminoalkyl, $C_{1-4}$-Cyanoalkyl oder $C_{1-4}$-Alkoxy-$C_{1-4}$-alkyl,

mit der Maßgabe, dass mindestens eine der Gruppen $R_{22}$ oder $R_{23}$ Wasserstoff bedeutet.

**8.** Zusammensetzung nach Anspruch 7, **dadurch gekennzeichnet, dass** die p-Aminophenole der Formel (V) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalzen mit einer Säure ausgewählt sind.

**9.** Zusammensetzung nach Anspruch 2, **dadurch gekennzeichnet, dass** die o-Aminophenole unter 2-Amino-phenol, 2-Amino-5-methylphenol, 2-Amino-6-methyl-phenol, 5-Acetamido-2-amino-phenol und deren Additionssalzen mit einer Säure ausgewählt sind.

**10.** Zusammensetzung nach Anspruch 2, **dadurch gekennzeichnet, dass** die heterocyclischen Oxidationsbasen unter den Pyridinderivaten, Pyrimidinderivaten, Pyrazolderivaten und deren Additionssalzen mit einer Säure ausgewählt sind.

**11.** Zusammensetzung nach einem der vorhergehenden Ansprüche, **dadurch gekennzeichnet, dass** die Oxidationsbase(n) 0,0005 bis 12 Gew.-% des Gesamtgewichts der Farbmittelzusammensetzung ausmachen.

**12.** Zusammensetzung nach Anspruch 11, **dadurch gekennzeichnet, dass** die Oxidationsbase(n) 0,005 bis 6 Gew.-% des Gesamtgewichts der Farbmittelzusammensetzung ausmachen.

**13.** Zusammensetzung nach einem der vorhergehenden Ansprüche, **dadurch gekennzeichnet, dass** das (die) 3-Amino-pyridinderivat(e) der Formel (I) unter den folgenden Verbindungen ausgewählt ist (sind):

- 3-[4'-Dimethylamino-1'-benzolazo]-1-methyl-pyridiniummethosulfat der folgenden Formel:

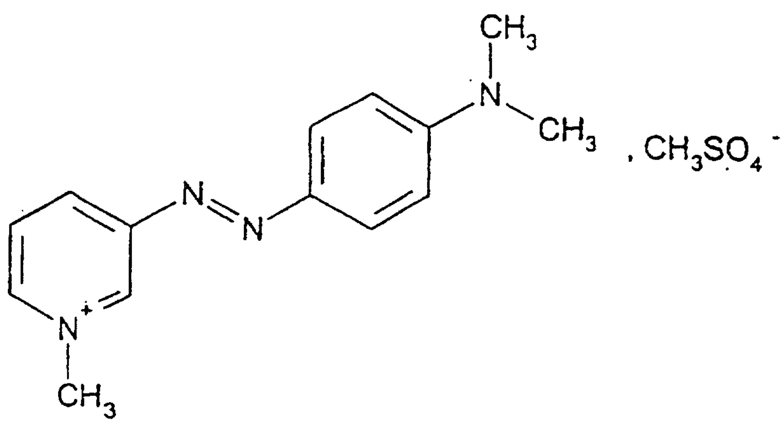

- 3-[4'-Bis(β-hydroxyethyl)amino-1'-benzolazo]-1-methyl-pyridiniummethosulfat der folgenden Formel:

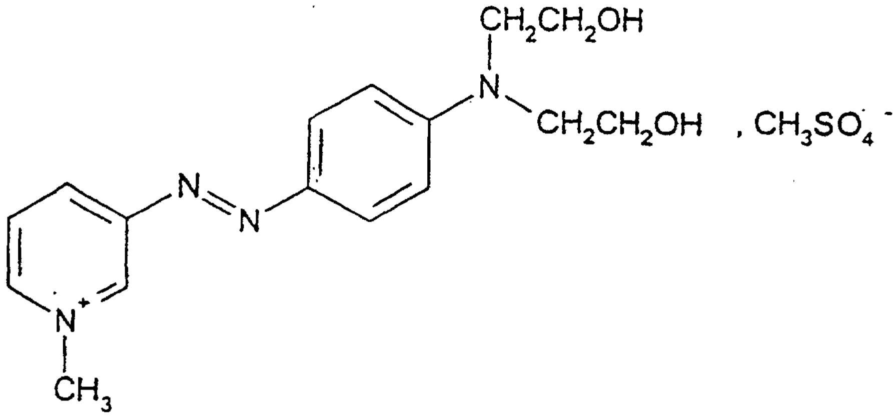

- 3-[4'-Amino-8'-hydroxy-1'-naphthalinazo]-1-methyl-pyridiniummethosulfat der folgenden Formel:

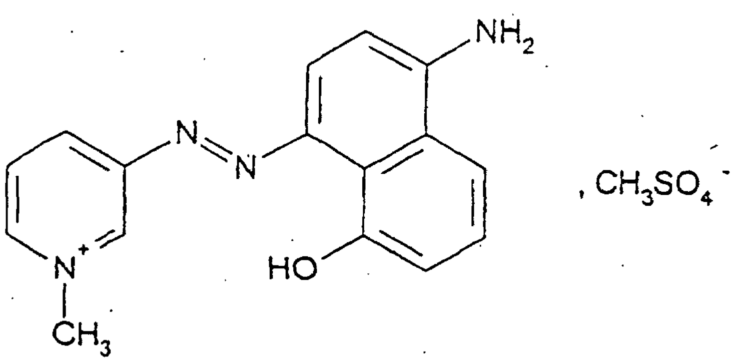

- 3-[4'-Dimethylamino-2'-nitro-1'-benzolazo]-1-methyl-pyridiniummethosulfat der folgenden Formel:

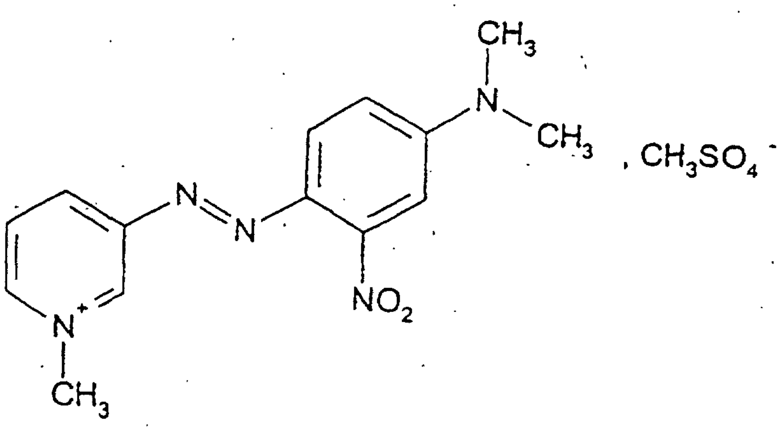

- 3-[4'-Dimethylamino-1'-benzolazo]-1,6-dimethyl-pyridiniummethosulfat der folgenden Formel:

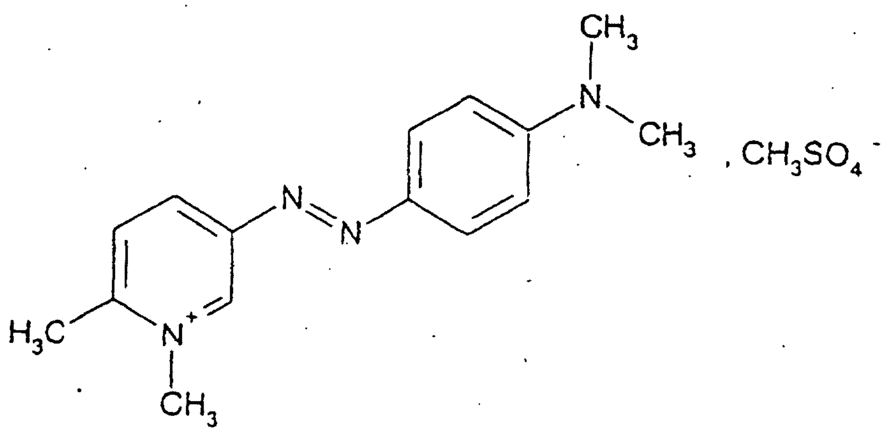

- 3-[4'-Amino-1'-benzolazo]-pyridin-N-oxid der folgenden Formel:

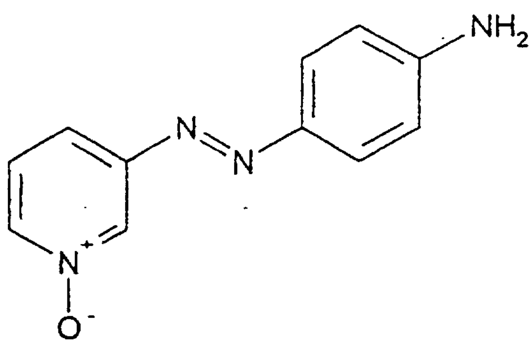

- 3-[4'-Dimethylamino-1'-benzolazo]-pyridin-N-oxid der folgenden Formel:

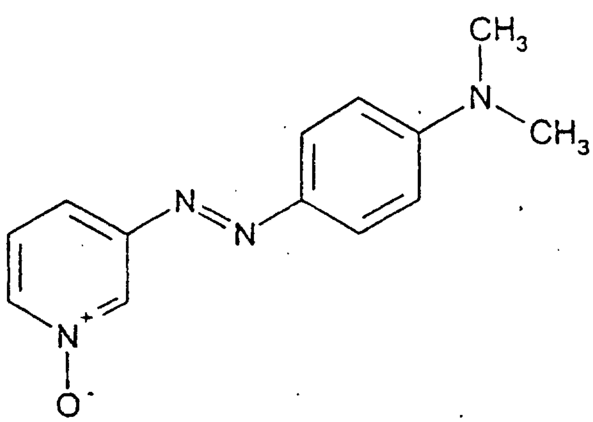

- 3-[4'-N,N-Bis(β-hydroxyethyl)amino-1'-benzolazo]-pyridin-N-oxid der folgenden Formel:

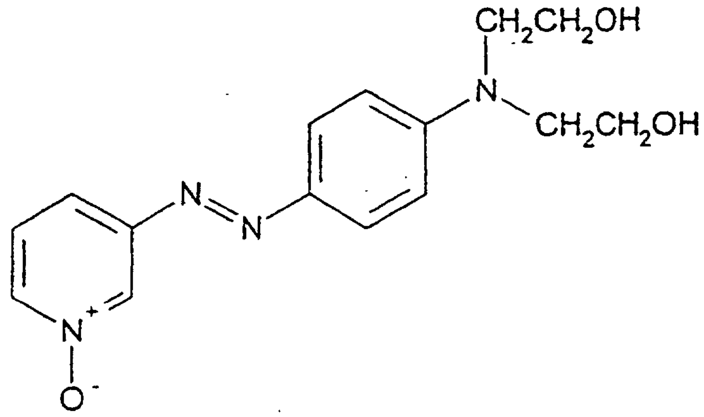

- 3-[4'-Dimethylamino-2'-methyl-1'-benzolazo]-1-ethyl-pyridiniumethosulfat der folgenden Formel:

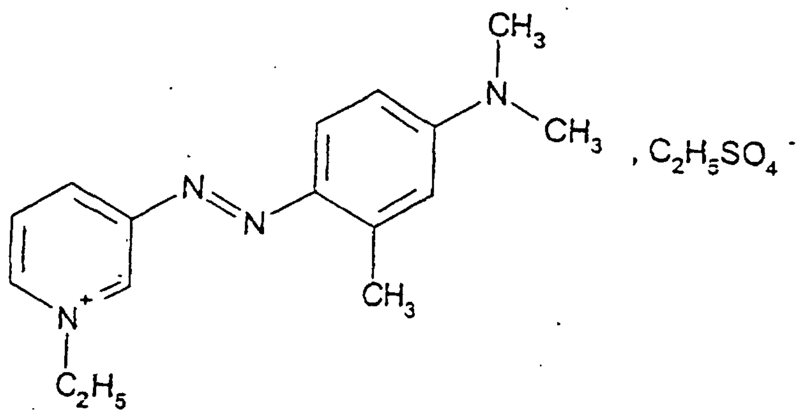

- 3-[4'-Dimethylamino-2'-methyl-1'-benzolazo]-1-butyl-pyridiniumbromid der folgenden Formel:

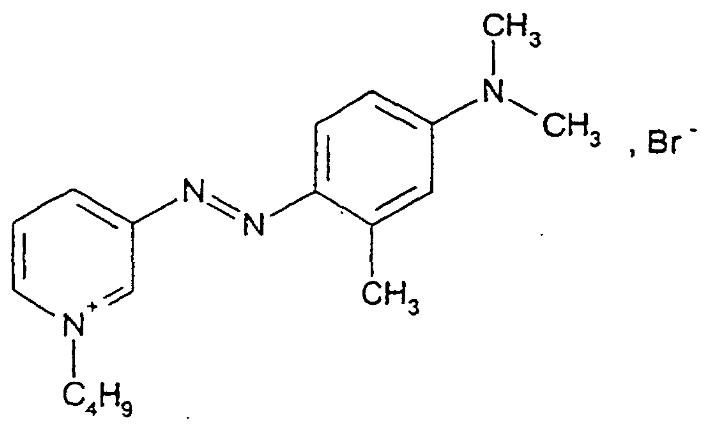

- 3-[4'-Dimethylamino-2'-chlor-1'-benzolazo]-1-methyl-pyridiniummethosulfat der folgenden Formel:

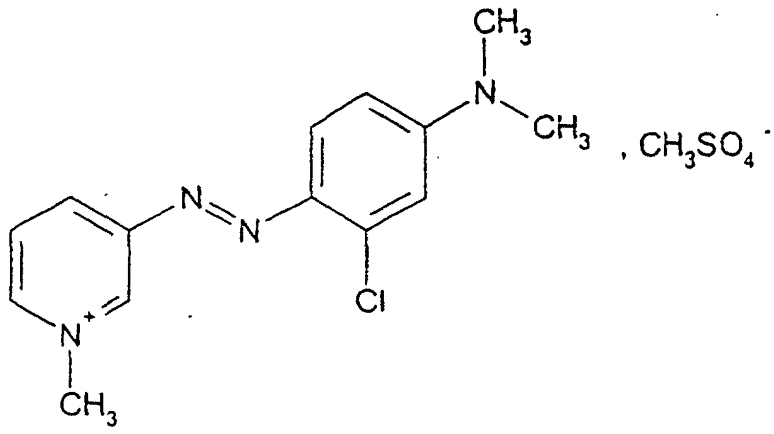

- 3-[2',4'-Diamino-5'-methyl-1'-benzolazo]-1-methyl-pyridiniummethosulfat der folgenden Formel:

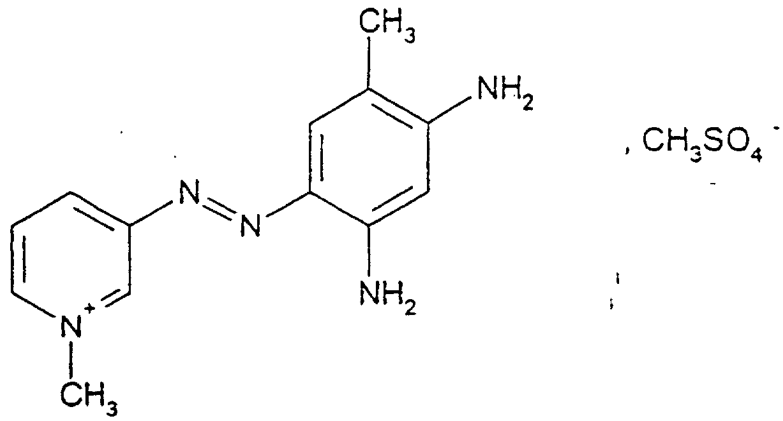

- 3-[4'-Phenylamino-1'-benzolazo]-1-methyl-pyridinium-methosulfat der folgenden Formel:

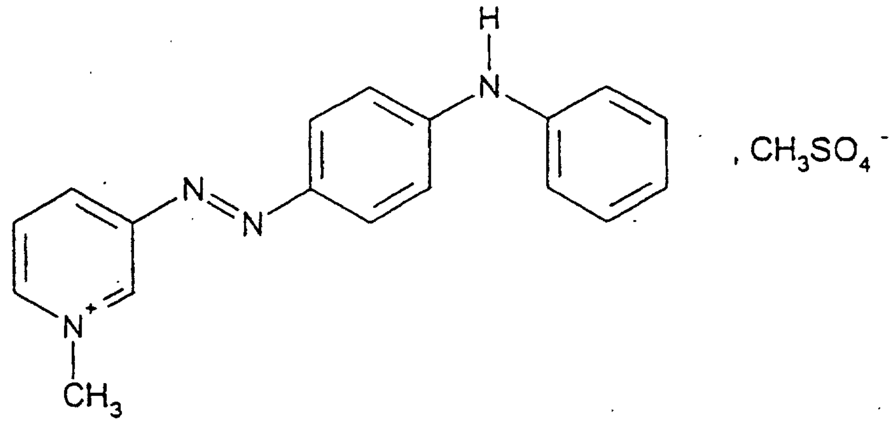

- 3-[2'-Acetylamino-4'-dimethylamino-1'-benzolazo]-1-ethylpyridinium-ethosulfat der folgenden Formel:

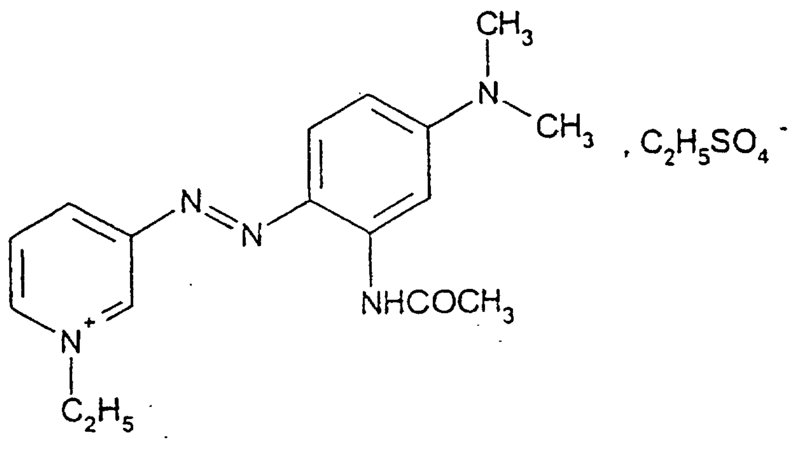

- 3-[2',4'-Diamino-5'-methoxy-1'-benzolazo]-1-methyl-pyridiniummethosulfat der folgenden Formel:

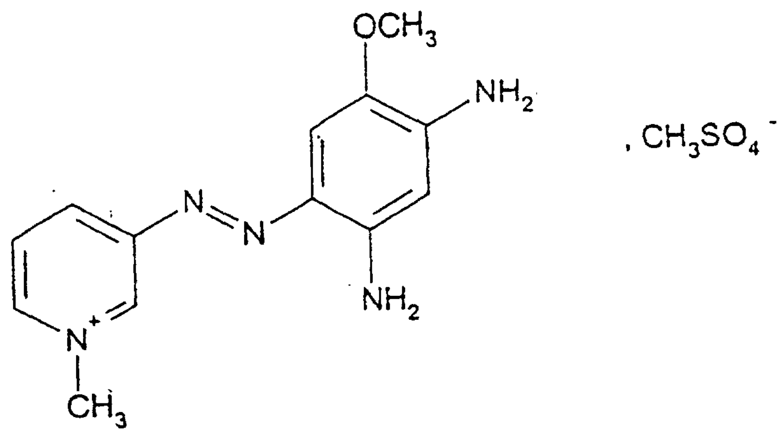

- 3-[2'-Amino-4'-Dimethylamino-1'-benzolazo]-1-methyl-pyridiniummethosulfat der folgenden Formel:

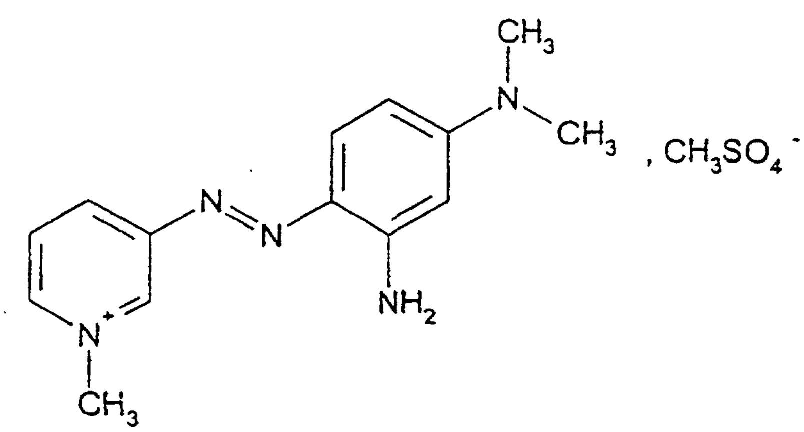

**14.** Zusammensetzung nach einem der vorhergehenden Ansprüche, **dadurch gekennzeichnet, dass** das (die) 3-Amino-pyridinderivat(e) der Formel (I) 0,001 bis 10 Gew.-% des Gesamtgewichts der Farbmittelzusammensetzung ausmachen.

**15.** Zusammensetzung nach Anspruch 14, **dadurch gekennzeichnet, dass** das (die) 3-Amino-pyridinderivat(e) der Formel (I) 0,01 bis 5 Gew.-% des Gesamtgewichts der Farbmittelzusammensetzung ausmachen.

**16.** Zusammensetzung nach einem der vorhergehenden Ansprüche, **dadurch gekennzeichnet, dass** die m-Aminophenolderivate der Formel (II) unter 5-Amino-2-methoxy-phenol, 5-Amino-2-(β-hydroxyethyloxy)-phenol, 5-Amino-2-methyl-phenol, 5-N-(β-Hydroxyethyl)amino-2-methyl-phenol, 5-N-(β-Hydroxyethyl)amino-4-methoxy-2-methyl-phenol, 5-Amino-4-methoxy-2-methyl-phenol, 5-Amino-4-chlor-2-methyl-phenol, 5-Amino-2,4-dimethoxy-phenol, 5-(γ-Hydroxypropylamino)-2-methyl-phenol, 3-Amino-2-chlor-6-methyl-phenol, 3-Amino-6-chlor-phenol, 3-(β-Aminoethyl)amino-6-chlor-phenol und den Additionssalzen dieser Verbindungen mit einer Säure ausgewählt sind.

**17.** Zusammensetzung nach einem der vorhergehenden Ansprüche, **dadurch gekennzeichnet, dass** das (die) m-Aminophenolderivat(e) der Formel (II) 0,0001 bis 10 Gew.-% des Gesamtgewichts der Farbmittelzusammensetzung ausmachen.

**18.** Zusammensetzung nach Anspruch 17, **dadurch gekennzeichnet, dass** das (die) m-Aminophenolderivat(e) der Formel (II) 0,005 bis 5 Gew.-% des Gesamtgewichts der Farbmittelzusammensetzung ausmachen.

**19.** Zusammensetzung nach einem der vorhergehenden Ansprüche, **dadurch gekennzeichnet, dass** sie einen oder mehrere Kuppler, die von den m-Aminophenolderivaten der Formel (II) nach Anspruch 1 verschieden sind, und/oder einen oder mehrere Direktfarbstoffe, die von den 3-Amino-pyridinderivaten der Formel (1) nach Anspruch 1 verschieden sind, enthält.

**20.** Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

**21.** Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

**22.** Zusammensetzung nach einem der vorhergehenden Ansprüche, **dadurch gekennzeichnet, dass** sie einen pH-Wert im Bereich von 3 bis 12 aufweist.

**23.** Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 22 aufgebracht wird, wobei die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

**24.** Verfahren nach Anspruch 23, **dadurch gekennzeichnet, dass** das Oxidationsmittel unter Wasserstoffperoxid, Harnstoffperoxid, Alkalimetallbromaten, Salzen von Persäuren, wie Perboraten, Percarbonaten und Persulfaten, Persäuren und Enzymen ausgewählt ist.

**25.** Vorrichtung mit mehreren Abteilungen oder "Kit" zum Färben mit mehreren Abteilungen, wobei eine erste Abteilung eine Farbmittelzusammensetzung nach einem der Ansprüche 1 bis 22 und eine zweite Abteilung eine oxidierende Zusammensetzung enthält.

## Claims

**1.** Composition for the oxidation dyeing of keratin fibres, and in particular of human keratin fibres such as the hair, **characterized in that** it comprises, in a medium which is suitable for dyeing:

- at least one oxidation base,
- as direct dye, at least one 3-aminopyridine derivative chosen from the compounds of formula (I) below:

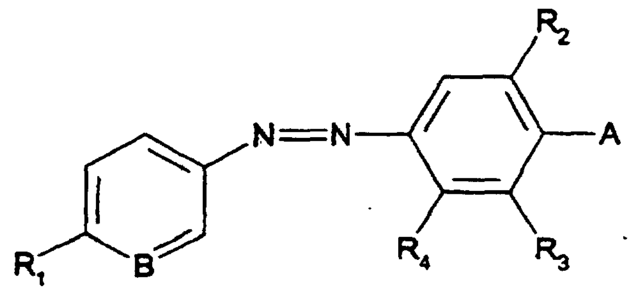

(I)

in which:

- B represents a group of formula (Ia) or (Ib) below:

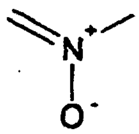

(Ia)

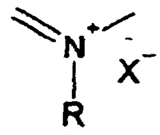

(Ib)

- R represents a $C_1$-$C_4$ alkyl radical;
- $R_1$ represents a hydrogen or halogen atom such as chlorine, bromine or fluorine, or a $C_1$-$C_4$ alkyl or $C_1$-$C_4$ alkoxy radical;
- $R_2$ represents a hydrogen atom or a $C_1$-$C_4$ alkyl or $C_1$-$C_4$ alkoxy radical;
- $R_4$ represents a hydrogen or halogen atom such as chlorine, bromine or fluorine, or a $C_1$-$C_4$ alkyl, nitro, amino or ($C_1$-$C_4$)acylamino radical;
- $R_3$ represents a hydrogen atom or else $R_4$ and $R_3$ together form a 6-membered unsaturated ring bearing a hydroxyl substituent chelated with one of the nitrogen atoms of the azo double bond;
- A represents a residue $-NR_5R_6$ in which $R_5$ represents a hydrogen atom or a $C_1$-$C_4$ alkyl, $C_1$-$C_4$ monohydroxyalkyl or $C_2$-$C_4$ polyhydroxyalkyl radical; $R_6$ represents a hydrogen atom, a $C_1$-$C_4$ alkyl, $C_1$-$C_4$ monohydroxyalkyl or $C_2$-$C_4$ polyhydroxyalkyl radical, a phenyl ring or a $-CH_2-SO_3Na$ radical;
- $X^-$ represents a monovalent or divalent anion and is preferably chosen from a halogen atom such as chlorine, bromine, fluorine or iodine, a hydroxide, a hydrogen sulphate or a ($C_1$-$C_6$)alkyl sulphate such as, for example, a methyl sulphate or an ethyl sulphate, and
- at least one coupler chosen from the meta-aminophenol derivatives of formula (II) below, and the addition salts thereof with an acid:

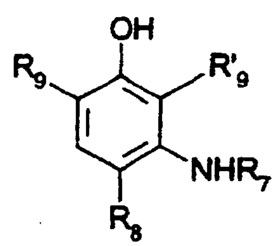

(II)

in which:

- $R_7$ represents a hydrogen atom or a $C_1$-$C_4$ alkyl, $C_1$-$C_4$ monohydroxyalkyl, $C_2$-$C_4$ polyhydroxyalkyl or $C_1$-$C_4$ monoaminoalkyl radical;
- $R_8$ represents a hydrogen atom, a $C_1$-$C_4$ alkyl or $C_1$-$C_4$ alkoxy radical or a halogen atom chosen from chlorine, bromine and fluorine,
- $R_9$ and $R'_9$, which may be identical or different, represent a hydrogen or halogen atom or a $C_1$-$C_4$ alkyl, $C_1$-$C_4$ alkoxy, $C_1$-$C_4$ monohydroxyalkyl, $C_2$-$C_4$ polyhydroxyalkyl, $C_1$-$C_4$ monohydroxyalkoxy or $C_2$-$C_4$ polyhydroxyalkoxy radical;

it being understood that at least one of the radicals $R_7$, $R_8$, $R_9$ and $R'_9$ is other than a hydrogen atom.

**2.** Composition according to Claim 1, **characterized in that** the oxidation base(s) is (are) chosen from para-phenylenediamines, double bases, para-aminophenols, ortho-aminophenols and heterocyclic oxidation bases.

**3.** Composition according to Claim 2, **characterized in that** the para-phenylenediamines are chosen from the compounds of formula (III) below, and the addition salts thereof with an acid:

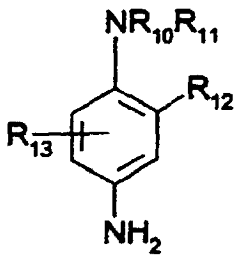

(III)

in which:

- $R_{10}$ represents a hydrogen atom or a $C_1$-$C_4$ alkyl radical, $C_1$-$C_4$ monohydroxyalkyl radical, $C_2$-$C_4$ polyhydroxyalkyl radical, ($C_1$-$C_4$)alkoxy($C_1$-$C_4$)alkyl radical, $C_1$-$C_4$ alkyl radical substituted with a nitrogenous group, phenyl or 4'-aminophenyl;
- $R_{11}$ represents a hydrogen atom or a $C_1$-$C_4$ alkyl radical, $C_1$-$C_4$ monohydroxyalkyl radical, $C_2$-$C_4$ polyhydroxyalkyl radical, ($C_1$-$C_4$)alkoxy ($C_1$-$C_4$)alkyl radical or $C_1$-$C_4$ alkyl radical substituted with a nitrogenous group;
- $R_{12}$ represents a hydrogen atom, a halogen atom such as a chlorine, bromine, iodine or fluorine atom, or a $C_1$-$C_4$ alkyl radical, $C_1$-$C_4$ monohydroxyalkyl radical, $C_1$-$C_4$ hydroxyalkoxy radical, acetylamino($C_1$-$C_4$)alkoxy, mesylamino($C_1$-$C_4$)alkoxy or carbamoylamino($C_1$-$C_4$)alkoxy radical,
- $R_{13}$ represents a hydrogen or halogen atom or a $C_1$-$C_4$ alkyl radical.

**4.** Composition according to Claim 3, **characterized in that** the para-phenylenediamines of formula (III) are chosen from para-phenylenediamine, paratolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine and N-(β-methoxyethyl)-para-phenylenediamine, and the addition salts thereof with an acid.

**5.** Composition according to Claim 2, **characterized in that** the double bases are chosen from the compounds of formula (IV) below, and the addition salts thereof with an acid:

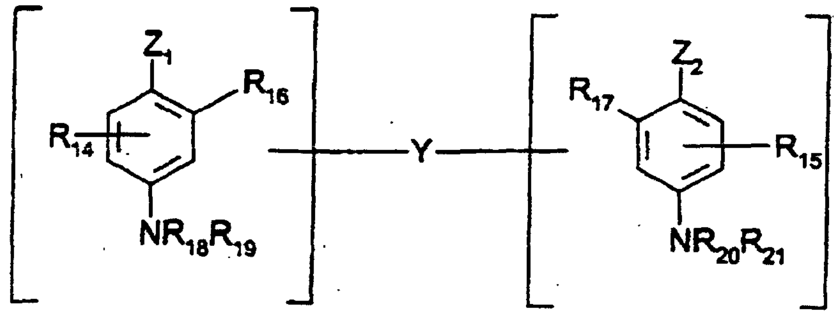

(II)

in which:

- $Z_1$ and $Z_2$, which may be identical or different, represent a hydroxyl or $-NH_2$ radical which can be substituted with a $C_1$-$C_4$ alkyl radical or with a linker arm Y;
- the linker arm Y represents a linear or branched alkylene chain comprising from 1 to 14 carbon atoms, which can be interrupted or terminated with one or more nitrogenous groups and/or with one or more hetero atoms such as oxygen, sulphur or nitrogen atoms, and optionally substituted with one or more hydroxyl or $C_1$-$C_6$ alkoxy radicals;
- $R_{14}$ and $R_{15}$ represent a hydrogen or halogen atom, a $C_1$-$C_4$ alkyl radical, $C_1$-$C_4$ monohydroxyalkyl radical, $C_2$-$C_4$ polyhydroxyalkyl radical or $C_1$-$C_4$ aminoalkyl radical or a linker arm Y;
- $R_{16}$, $R_{17}$, $R_{18}$, $R_{19}$, $R_{20}$ and $R_{21}$, which may be identical or different, represent a hydrogen atom, a linker arm Y or a $C_1$-$C_4$ alkyl radical;

it being understood that the compounds of formula (IV) comprise only one linker arm Y per molecule.

**6.** Composition according to Claim 5, **characterized in that** the double bases of formula (IV) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis (4-aminophenyl)-tetramethylenediamine, N,N'-bis(4-methylaminophenyl)tetramethylenediamine, N,N'-bis(ethyl) -N,N' -bis(4'-amino-3'-methylphenyl)ethylenediamine and 1,8-bis(2,5-diaminophenoxy)-3,5-dioxaoctane, and the addition salts thereof with an acid.

**7.** Composition according to Claim 2, **characterized in that** para-aminophenols are chosen from the compounds of formula (V) below, and the addition salts thereof with an acid:

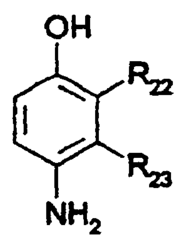

(V)

in which:

- $R_{22}$ represents a hydrogen or halogen atom or a $C_1$-$C_4$ alkyl, $C_1$-$C_4$ monohydroxyalkyl, ($C_1$-$C_4$)alkoxy ($C_1$-$C_4$) alkyl, $C_1$-$C_4$ aminoalkyl or hydroxy ($C_1$-$C_4$) alkylamino ($C_1$-$C_4$)alkyl radical,
- $R_{23}$ represents a hydrogen or halogen atom or a $C_1$-$C_4$ alkyl, $C_1$-$C_4$ monohydroxyalkyl, $C_2$-$C_4$ polyhydroxyalkyl, $C_1$-$C_4$ aminoalkyl, cyano($C_1$-$C_4$)alkyl or ($C_1$-$C_4$) alkoxy($C_1$-$C_4$)alkyl radical,

it being understood that at least one of the radicals $R_{22}$ and $R_{23}$ represents a hydrogen atom.

**8.** Composition according to Claim 7, **characterized in that** the para-aminophenols of formula (V) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

**9.** Composition according to Claim 2, **characterized in that** the ortho-aminophenols are chosen from 2-aminophenol, 2-amino-5-methylphenol, 2-amino-6-methylphenol and 5-acetamido-2-aminophenol, and the addition salts thereof with an acid.

**10.** Composition according to Claim 2, **characterized in that** the heterocyclic oxidation bases are chosen from pyridine derivatives, pyrimidine derivatives and pyrazole derivatives, and the addition salts thereof with an acid.

**11.** Composition according to any one of the preceding claims, **characterized in that** the oxidation base(s) represent (s) from 0.0005 to 12% by weight relative to the total weight of the dye composition.

**12.** Composition according to Claim 11, **characterized in that** the oxidation base(s) represent(s) from 0.005 to 6% by weight relative to the total weight of the dye composition.

**13.** Composition according to any one of the preceding claims, **characterized in that** the 3-aminopyridine derivative (s) of formula (I) is (are) chosen from:

- 4'-dimethylaminobenzene-1'-azo-1-methyl-3-pyridinium methosulphate of formula:

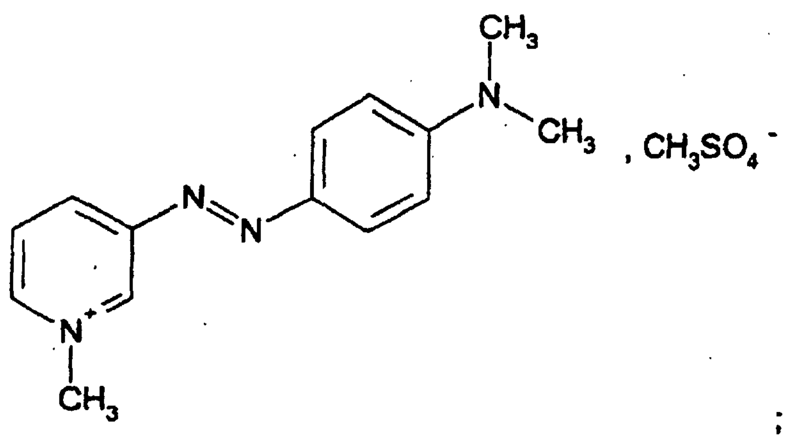

;

- 4'-bis(β-hydroxyethyl)aminobenzene-1'-azo-1-methyl-3-pyridinium methosulphate of formula:

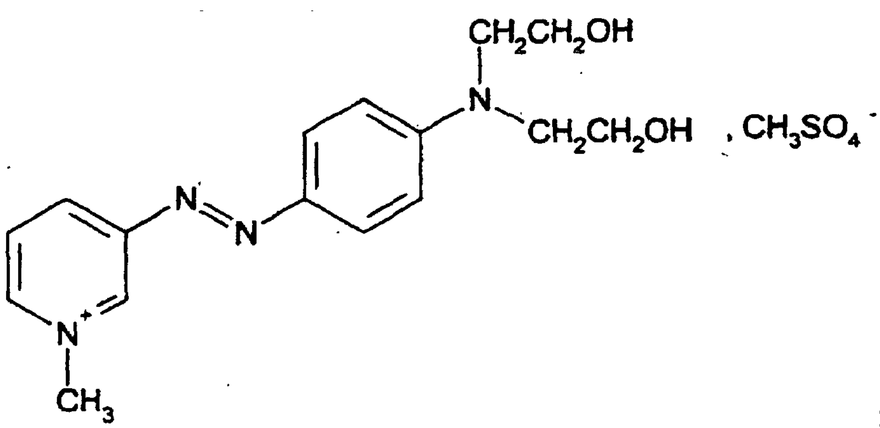

;

- 4'-amino-8'-hydroxynaphthalene-1'-azo-1-methyl-3-pyridinium methosulphate of formula:

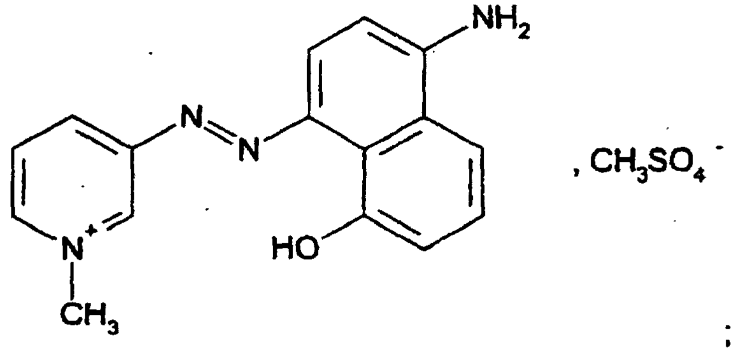

;

- 4'-dimethylamino-2'-nitrobenzene-1'-azo-1-methyl-3-pyridinium methosulphate of formula:

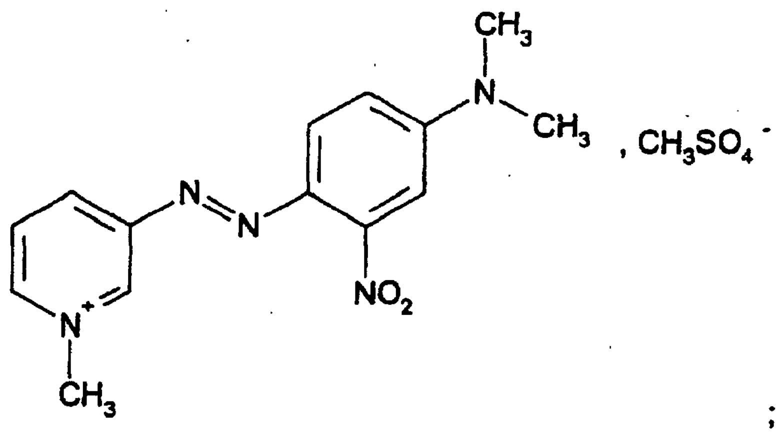

;

- 4'-dimethylaminobenzene-1'-azo-1,6-dimethyl-3-pyridinium methosulphate of formula:

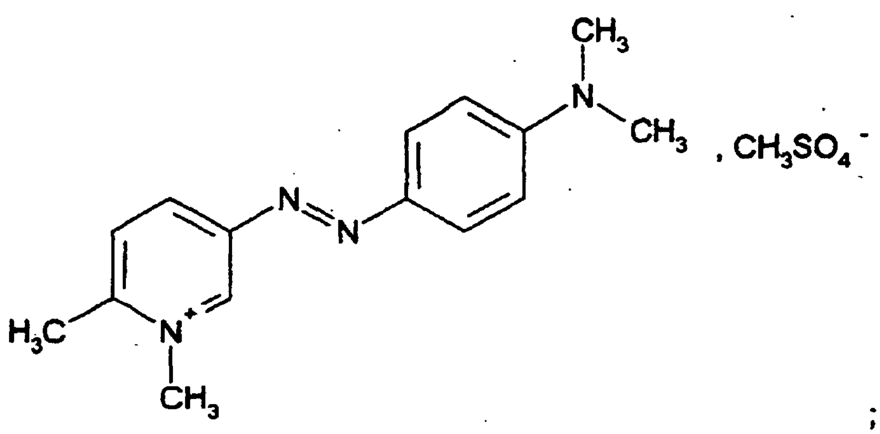

;

- 4'-aminobenzene-1'-azo-3-pyridine N-oxide of formula:

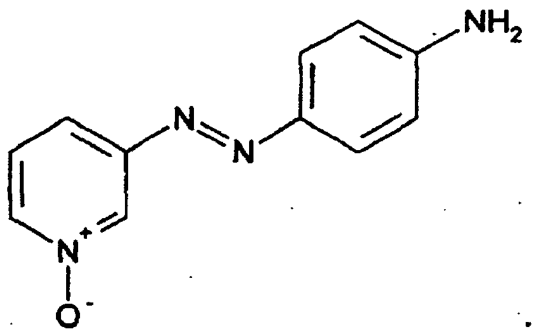

;

- 4'-dimethylaminobenzene-1'-azo-3-pyridine N-oxide of formula:

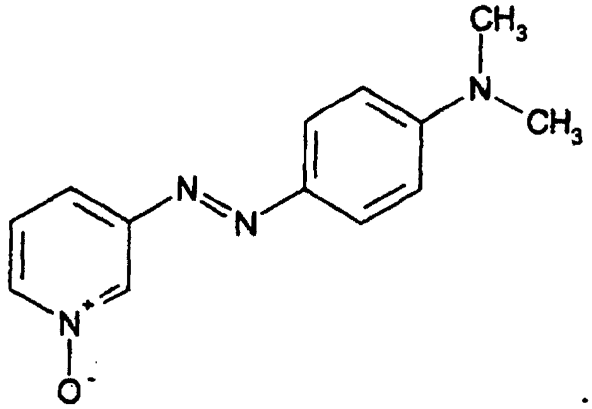

;

- 4'-N,N-bis(β-hydroxyethyl)aminobenzene-1'-azo-3-pyridine N-oxide of formula:

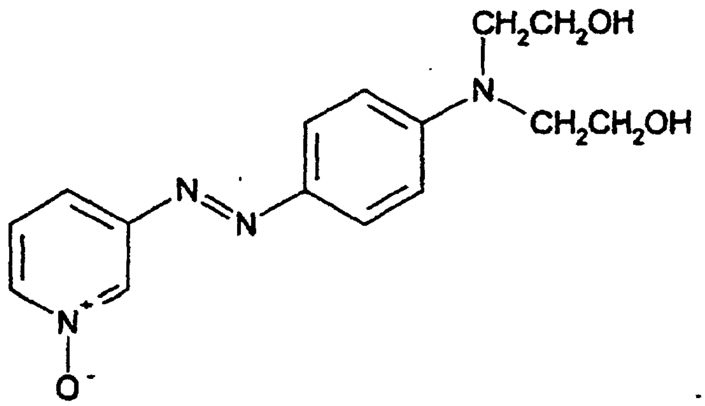

;

- 4'-dimethylamino-2'-methylbenzene-1'-azo-1-ethyl-3-pyridinium ethosulphate of formula :

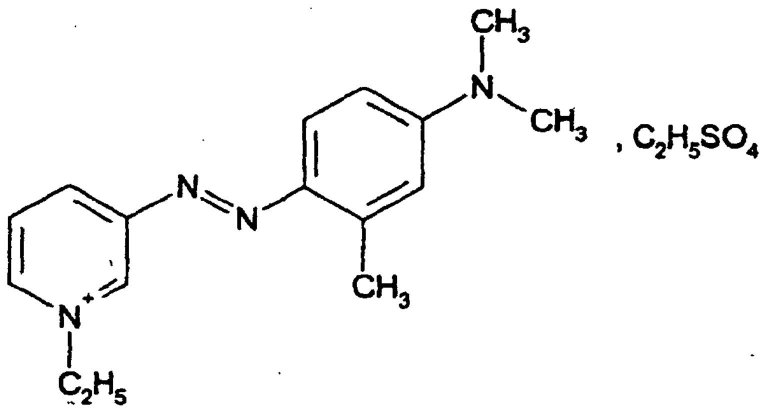

;

- 4'-dimethylamino-2'-methylbenzene-1'-azo-1-butyl-3-pyridinium bromide of formula:

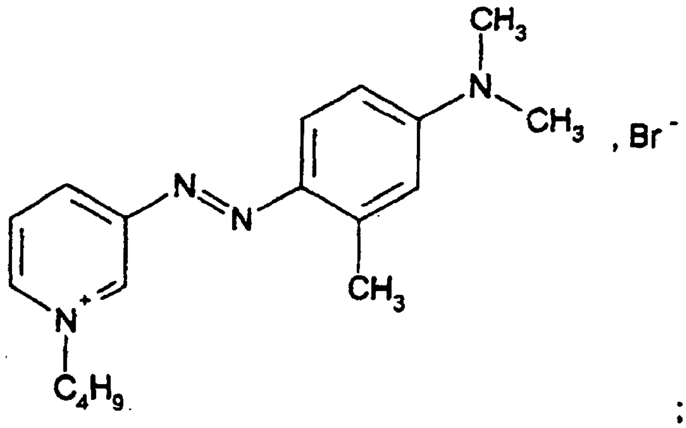

;

- 4'-dimethylamino-2'-chlorobenzene-1'-azo-1-methyl-3-pyridinium methosulphate of formula:

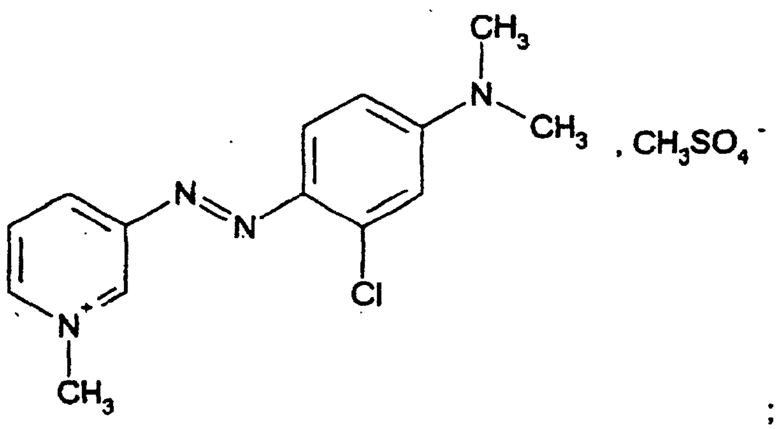

;

- 2',4'-diamino-5'-methylbenzene-1'-azo-1-methyl-3-pyridinium methosulphate of formula:

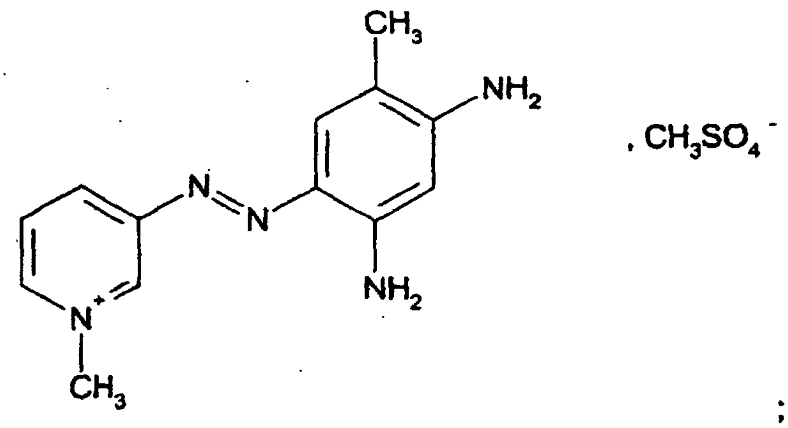

;

- 4'-phenylaminobenzene-1'-azo-1-methyl-3-pyridinium methosulphate of formula:

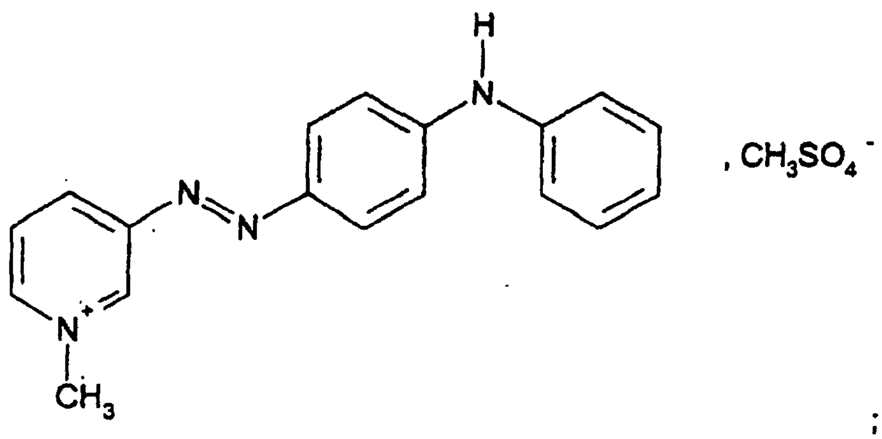

;

- 2'-acetylamino-4'-dimethylaminobenzene-1'-azo-1-ethyl-3-pyridinium ethosulphate of formula:

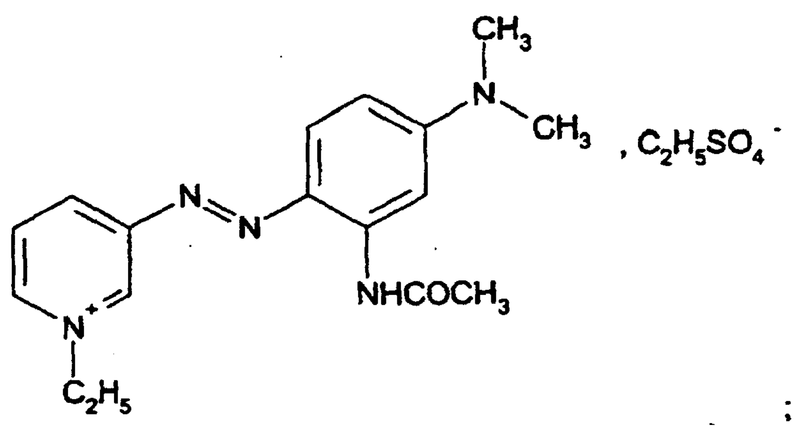

;

- 2',4'-diamino-5'-methoxybenzene-1'-azo-1-methyl-3-pyridinium methosulphate of formula:

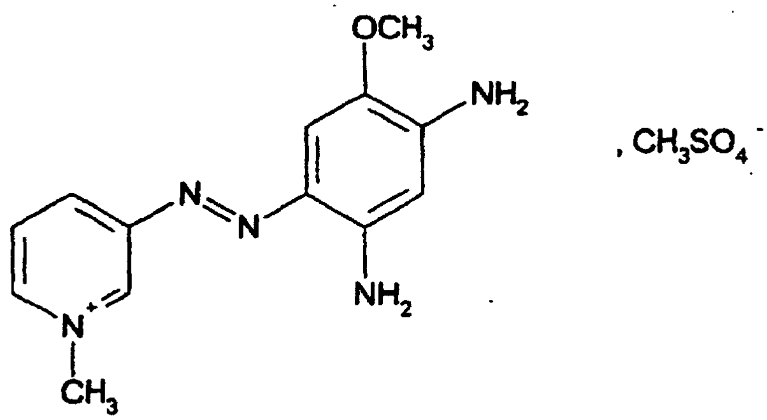

and

- 2'-amino-4'-dimethylaminobenzene-1'-azo-1-methyl-3-pyridinium methosulphate of formula:

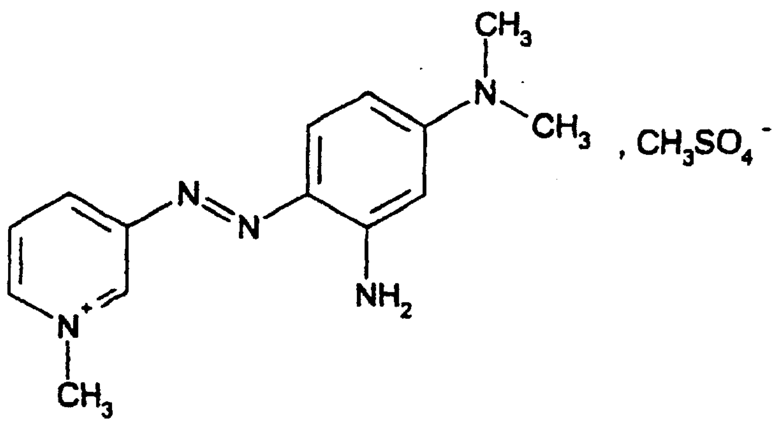

**14.** Composition according to any one of the preceding claims, **characterized in that** the 3-aminopyridine derivative (s) of formula (I) represent(s) from 0.001 to 10% by weight relative to the total weight of the dye composition.

**15.** Composition according to Claim 14, **characterized in that** the 3-aminopyridine derivative(s) of formula (I) represent (s) from 0.01 to 5% by weight relative to the total weight of the dye composition.

**16.** Composition according to any one of the preceding claims, **characterized in that** the meta-aminophenol derivatives of formula (II) are chosen from 5-amino-2-methoxyphenol, 5-amino-2-(β-hydroxyethyloxy)phenol, 5-amino-2-methylphenol, 5-N-(β-hydroxyethyl)amino-2-methylphenol, 5-N-(β-hydroxyethyl)amino-4-methoxy-2-methylphenol, 5-amino-4-methoxy-2-methylphenol, 5-amino-4-chloro-2-methylphenol, 5-amino-2,4-dimethoxyphenol, 5-(γ-hydroxypropylamino)-2-methylphenol, 3-amino-2-chloro-6-methylphenol, 3-amino-6-chlorophenol and 3-(β-aminoethyl)amino-6-chlorophenol, and the addition salts thereof with an acid.

**17.** Composition according to any one of the preceding claims, **characterized in that** the meta-aminophenol derivative (s) of formula (II) represent(s) from 0.0001 to 10% by weight relative to the total weight of the dye composition.

**18.** Composition according to Claim 17, **characterized in that** the meta-aminophenol derivative(s) of formula (II) represent(s) from 0.005 to 5% by weight relative to the total weight of the dye composition.

**19.** Composition according to any one of the preceding claims, **characterized in that** it contains one or more couplers other than the meta-aminophenol derivatives of formula (II) as defined in Claim 1 and/or one or more direct dyes other than the 3-aminopyridine derivatives of formula (I) as defined in Claim 1.

**20.** Composition according to any one of the preceding claims, **characterized in that** the addition salts with an acid are chosen from the hydrochlorides, hydrobromides, sulphates, tartrates, lactates and acetates.

**21.** Composition according to any one of the preceding claims, **characterized in that** the medium which is suitable for dyeing consists of water or of a mixture of water and at least one organic solvent.

**22.** Composition according to any one of the preceding claims, **characterized in that** it has a pH of between 3 and 12.

**23.** Process for dyeing keratin fibres, and in particular human keratin fibres such as the hair, **characterized in that** at least one dye composition as defined in any one of Claims 1 to 22 is applied to the said fibres, the colour being developed at acidic, neutral or alkaline pH with the aid of an oxidizing agent which is added to the dye composition only at the time of use, or which is present in an oxidizing composition that is applied simultaneously or sequentially.

**24.** Process according to Claim 23, **characterized in that** the oxidizing agent present in the oxidizing composition is chosen from hydrogen peroxide, urea peroxide, alkali metal bromates, persalts such as perborates, percarbonates and persulphates, peracids and enzymes.

**25.** Multi-compartment dyeing device or multi-compartment dyeing "kit", a first compartment of which contains a dye composition as defined in any one of Claims 1 to 22 and a second compartment of which contains an oxidizing composition.